(19) Europäisches Patentamt
European Patent Office
Office européen des brevets

(11) EP 4 437 963 B1

(12) **EUROPEAN PATENT SPECIFICATION**

(45) Date of publication and mention of the grant of the patent:
**01.07.2026 Bulletin 2026/27**

(21) Application number: **23202992.6**

(22) Date of filing: **11.10.2023**

(51) International Patent Classification (IPC):
***A61B 5/30*** *(2021.01)* ***A61B 5/00*** *(2006.01)*
***H03G 3/20*** *(2006.01)*

(52) Cooperative Patent Classification (CPC):
**A61B 5/30; A61B 5/72;** A61B 5/742; G16H 50/30

(54) **METHOD AND APPARATUS FOR PROCESSING HUMAN BIOLOGICAL SIGNAL DATA, DEVICE, AND STORAGE MEDIUM**

VERFAHREN UND VORRICHTUNG ZUR VERARBEITUNG VON DATEN MENSCHLICHER BIOLOGISCHER SIGNALE, VORRICHTUNG UND SPEICHERMEDIUM

PROCÉDÉ ET APPAREIL DE TRAITEMENT DE DONNÉES DE SIGNAL BIOLOGIQUE HUMAIN, DISPOSITIF ET SUPPORT DE STOCKAGE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.10.2022 CN 202211337784**

(43) Date of publication of application:
**02.10.2024 Bulletin 2024/40**

(73) Proprietor: **Kingfar International Inc.**
**100085 Beijing (CN)**

(72) Inventors:
- **ZHAO, Qichao**
  **Beijing, 100085 (CN)**
- **YANG, Ran**
  **Beijing, 100085 (CN)**

(74) Representative: **Dr. Gassner & Partner mbB**
**Wetterkreuz 3**
**91058 Erlangen (DE)**

(56) References cited:
**CN-A- 101 534 102**
**CN-A- 112 773 381**
**US-A1- 2008 075 304**
**US-A1- 2022 034 857**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 437 963 B1

## Description

### TECHNICAL FIELD

The present application relates to the technical field of medical detection, and in particular to a method and apparatus for processing human biological signal data, a device and a storage medium.

### BACKGROUND

Human biological signals, such as photoplethysmography (PPG) and electrocardiogram (ECG) signals, are generally collected through an analog front end in a hardware circuit, and the human biological signals are amplified to a reasonable range that can be collected by an analog-to-digital converter (ADC), and then the analog signals are digitized through the ADC and read into an MCU; and the MCU performs filtering processing on the signals, such as high-pass, lowpass, band-pass and band-stop filtering to remove irrelevant noise signals, such as power frequency and aliasing signals. Human biological signals such as PPG and ECG signals can be used for calculation of signal values of heart rate (HR) and respiratory rate (RPM).

For some people, the heartbeat is relatively weak, and the measured PPG and ECG signals will also be very weak, and the waveform changes are not obvious and the waveform features are unclear, which is not conducive to the observation of PPG and ECG signals, and the signal values of heart rate (hr) and respiratory rate (rpm) cannot be accurately calculated. However, for a person who is doing sports or has just finished doing sports, his heartbeat is relatively strong, and the measured PPG and ECG signals will also be very strong, and the waveform amplitude is too large, exceeding the display range of the equipment, resulting in incomplete waveform display. Since only part of the waveform can be displayed, the waveform features cannot be clearly displayed, which is not conducive to the observation of PPG and ECG signals, and cannot accurately calculate the signal values of heart rate (hr) and respiratory rate (rpm).

In order to completely and clearly display the waveform features of PPG and ECG signals, generally, weak signals are amplified directly, or strong signals are shrinked directly. However, if the original signal is instantaneously amplified or shrinked, the change between the signals before and after processing is very abrupt, leading to the problem of waveform abnormality, etc. CN 101 534 102 A discloses a method for processing human biological signal data and a corresponding device and computer program.

### SUMMARY

In order to solve the problem of abnormality occurred in adjustment of human biological signal data and improve the accuracy of data indicators and parameters, the present application provides a method and apparatus for processing human biological signal data, a device and a storage medium.

In a first aspect, the present application provides a method for processing human biological signal data, and the following technical solution is adopted.

A method for processing human biological signal data, wherein an original human biological signal is traversed with a plurality of preset peak-to-peak value adjustment time windows; and for the original human biological signal in any one of the plurality of peak-to-peak value adjustment time windows except a first peak-to-peak value adjustment time window, the any one of the peak-to-peak value adjustment time windows is taken as a current peak-to-peak value adjustment time window, and the method includes:

when a previous peak-to-peak value adjustment time window of the current peak-to-peak value adjustment time window ends, acquiring an original peak-to-peak value of the original human biological signal within the previous peak-to-peak value adjustment time window;
determining a target adjustment multiple of the current peak-to-peak value adjustment time window based on the original peak-to-peak value and a preset target peak-to-peak value;
determining a plurality of actual adjustment multiples of the current peak-to-peak value adjustment time window based on the target adjustment multiple, and sequentially adjusting signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value;
wherein the plurality of actual adjustment multiples correspond to the signal values at the plurality of sampling points one by one.

Optionally, the determining a plurality of actual adjustment multiples of the current peak-to-peak value adjustment time window based on the target adjustment multiple, and sequentially adjusting signal values of the original human biological signal at a plurality of sampling points within the current peak-to-peak value adjustment time window based on the plurality of actual adjustment multiples includes:

determining an initial multiple single-step variation based on the target adjustment multiple and a preset initial actual adjustment multiple before acquiring a signal value of the original human biological signal at a first sampling point within the current peak-to-peak value adjustment time window;
determining a second actual adjustment multiple based on the initial actual adjustment multiple and the initial multiple single-step variation, and taking the second actual adjustment multiple as a current actual adjustment multiple;
adjusting the signal value at the first sampling point based on the current actual adjustment multiple; and
determining a next multiple single-step variation based on the target adjustment multiple and the current actual adjustment multiple, determining a next actual adjustment multiple based on the current actual adjustment multiple and the next multiple single-step variation, adjusting a signal value at a next sampling point based on the next actual adjustment multiple, then taking the next actual adjustment multiple as a current actual adjustment multiple, and repeating the step of determining a next multiple single-step variation based on the target adjustment multiple and the current actual adjustment multiple until at least one first preset condition is satisfied;
wherein the first preset condition includes:

the current actual adjustment multiple belongs to a first target interval, wherein the first target interval is determined by the target adjustment multiple; and
a window end time of the current peak-to-peak value adjustment time window is reached.

Optionally, if the current actual adjustment multiple belongs to the first target interval and the window end time of the current peak-to-peak value adjustment time window is not reached, signal values of the original human biological signal at the remaining sampling points within the current peak-to-peak value adjustment time window are adjusted based on the current actual adjustment multiple until the window end time of the current peak-to-peak value adjustment time window is reached.
Optionally, the plurality of actual adjustment multiples exhibit an increasing trend over time.
In a second aspect, the present application provides a method for processing human biological signal data, and the following technical solution is adopted.
A method for processing human biological signal data, wherein an original human biological signal is traversed with a plurality of preset baseline adjustment time windows; and for the original human biological signal in any one of the plurality of baseline adjustment time windows except a first baseline adjustment time window, the any one of the baseline adjustment time windows is taken as a current baseline adjustment time window, and the method includes:

when a previous baseline adjustment time window of the current baseline adjustment time window ends, acquiring an original baseline value of the original human biological signal within the previous baseline adjustment time window;
determining a target offset of the current baseline adjustment time window based on the original baseline value and a preset target baseline value; and
determining a plurality of actual offsets of the current baseline adjustment time window based on the target offset, and sequentially offsetting a baseline of the original human biological signal at a plurality of sampling points within the current baseline adjustment time window based on the plurality of actual offsets, so that the baseline of the original human biological signal within the current baseline adjustment time window gradually approaches the target baseline value;
wherein the plurality of actual offsets correspond to the baseline at the plurality of sampling points one by one.

Optionally, the determining a plurality of actual offsets of the current baseline adjustment time window based on the target offset, and sequentially offsetting a baseline of the original human biological signal at a plurality of sampling points within the current baseline adjustment time window based on the plurality of actual offsets includes:

determining an initial baseline single-step variation based on the target baseline value and a preset initial actual offset before acquiring the baseline of the original human biological signal at a first sampling point within the current baseline adjustment time window;
determining a second actual offset based on the initial actual offset and the initial baseline single-step variation, and taking the second actual offset as a current actual offset;
offsetting the baseline at the first sampling point based on the current actual offset; and
determining a next baseline single-step variation based on the target offset and the current actual offset, determining a next actual offset based on the current actual offset and the next baseline single-step variation, offsetting the baseline at a next sampling point based on the next actual offset, then taking the next actual offset as a current actual offset, and repeating the step of determining a next baseline single-step variation based on the target offset and the current actual offset until at least one second preset condition is satisfied;
wherein the second preset condition includes:

the current actual offset belongs to a second target interval, wherein the second target interval is determined by the target offset; and
a window end time of the current baseline adjustment time window is reached.

If the current actual offset belongs to the second target interval and the window end time of the current baseline adjustment time window is not reached, the baseline of the original human biological signal at the remaining sampling points within the current baseline adjustment time window is offset based on the current actual offset until the window end time of the current baseline adjustment time window is reached.
In a third aspect, the present application provides an apparatus for processing human biological signal data, and the following technical solution is adopted.
An apparatus for processing human biological signal data, wherein:
an original human biological signal is traversed with a plurality of preset peak-to-peak value adjustment time windows; and for the original human biological signal in any one of the plurality of peak-to-peak value adjustment time windows except a first peak-to-peak value adjustment time window, the any one of the peak-to-peak value adjustment time windows is taken as a current peak-to-peak value adjustment time window, and the apparatus includes:

a first acquisition module configured to, when a previous peak-to-peak value adjustment time window of the current peak-to-peak value adjustment time window ends, acquire an original peak-to-peak value of the original human biological signal within the previous peak-to-peak value adjustment time window;
a first determination module configured to determine a target adjustment multiple of a current peak-to-peak value adjustment time window based on the original peak-to-peak value and a preset target peak-to-peak value; and
an adjustment module configured to determine a plurality of actual adjustment multiples of the current peak-to-peak value adjustment time window based on the target adjustment multiples, and sequentially adjusting signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value; wherein the plurality of actual adjustment multiples correspond to the signal values at the plurality of sampling points one by one.

In a fourth aspect, the present application provides an apparatus for processing human biological signal data, and the following technical solution is adopted.
An apparatus for processing human biological signal data, wherein an original human biological signal is traversed with a plurality of preset baseline adjustment time windows; and for the original human biological signal in any one of the plurality of baseline adjustment time windows except a first baseline adjustment time window, the any one of the baseline adjustment time windows is taken as a current baseline adjustment time window, and the apparatus includes:

a second acquisition module configured to, when a previous baseline adjustment time window of the current baseline adjustment time window ends, acquire an original baseline value of the original human biological signal within the previous baseline adjustment time window;
a second determination module configured to determine a target offset of the current baseline adjustment time window based on the original baseline value and a preset target baseline value; and
an offsetting module configured to determine a plurality of actual offsets of the current baseline adjustment time window based on the target offset, and sequentially offsetting a baseline of the original human biological signal at a plurality of sampling points within the current baseline adjustment time window based on the plurality of actual offsets, so that the baseline of the original human biological signal within the current baseline adjustment time window gradually approaches the target baseline value; wherein the plurality of actual offsets correspond to the baseline at the plurality of sampling points one by one.

In a fifth aspect, the present application provides an electronic device, and the following technical solution is adopted.
An electronic device includes a memory and a processor; wherein the memory has stored thereon a computer program to be loaded by the processor to perform the method for any item of the first aspect.
In a sixth aspect, the present application provides a computer-readable storage medium, and the following technical solution is adopted.

A computer readable storage medium has stored thereon a computer program to be loaded by a processor to perform the method for any item of the first or second aspects.

By using the above-mentioned technical solution, the peak-to-peak value of the original human biological signal, which is weak or too strong, can be gradually adjusted to a suitable degree with the display area of the waveform display device, and the baseline of the original human biological signal can also be gradually adjusted to a suitable position in the display area of the waveform display device to clearly and completely display the waveform features of the human biological signal, and the situation of waveform distortion of the human biological signal caused by directly adjusting the peak-to-peak value and the baseline can be improved.

## BRIEF DESCRIPTION OF THE DRAWINGS

FIG. 1 is a schematic diagram of traversing an original human biological signal with a plurality of peak-to-peak value adjustment time windows according to an embodiment of the present application.

FIG. 2 is a schematic flowchart of a method for processing human biological signal data according to an embodiment of the present application.

FIG. 3 is a schematic flowchart of sub-steps of Step S103 in a method for processing human biological signal data according to an embodiment of the present application.

FIG. 4 is a waveform diagram for processing a weak original human biological signal using a method for processing human biological signal data according to an embodiment of the present application.

FIG. 5 is a waveform diagram for processing an excessively strong original human biological signal using a method for processing human biological signal data according to an embodiment of the present application.

FIG. 6 is a schematic flowchart of another method for processing human biological signal data according to an embodiment of the present application.

FIG. 7 is a flowchart showing sub-steps of Step S203 in another method for processing human biological signal data according to an embodiment of the present application.

FIG. 8 is a waveform diagram for adjusting a baseline of an original human biological signal using a method for processing human biological signal data according to an embodiment of the present application.

FIG. 9 is a block diagram of an apparatus for processing human biological signal data according to an embodiment of the present application.

FIG. 10 is a block diagram of another apparatus for processing human biological signal data according to an embodiment of the present application.

FIG. 11 is a block diagram of an electronic device according to an embodiment of the present application.

## DETAILED DESCRIPTION

In order that the objects, aspects and advantages of the embodiments of the present invention will become more apparent, a more complete description of the embodiments of the present invention will be rendered by reference to the appended drawings.

In order to solve the problem of waveform distortion caused by the instantaneous amplification or reduction of a signal, the embodiment of the present application needs to realize the step-by-step amplification or reduction of an original human biological signal; therefore, a plurality of peak-to-peak value adjustment time windows are used to perform traversal sampling on the original human biological signal, i.e., the original human biological signal is segmented according to time, and the original human biological signal within each peak-to-peak value adjustment time window is processed successively.

As shown in FIG. 1, for two adjacent peak-to-peak value adjustment time windows, the end time of the previous peak-to-peak value adjustment time window is the start time of the next peak-to-peak value adjustment time window. At the end of each peak-to-peak value adjustment time window, the maximum value and minimum value of the original human biological signal within the peak-to-peak value adjustment time window would be calculated, stored into a buffer with fixed length, performed sliding median filtering on the maximum value and minimum value stored in the buffer, the window mark is cleared, and the next peak-to-peak value adjustment time window is ready.

In the embodiments of the present application, the step-by-step adjustment of the signal by each peak-to-peak value adjustment time window is performed on the basis of the peak-to-peak value of the signal of the previous peak-to-peak value adjustment time window; therefore, this embodiment abandons the signal processing of the first peak-to-peak value adjustment time window, and the first peak-to-peak value adjustment time window merely provides a peak-to-peak value for the second peak-to-peak value adjustment time window. In addition, the waveform processing method for the original human biological signal of any peak-to-peak value adjustment time window except the first peak-to-peak value adjustment time window is the same.

The waveform processing method for the original human biological signal of any peak-to-peak value adjustment time window except the first peak-to-peak value adjustment time window is specifically described below, wherein any peak-to-peak value adjustment time window is used as the current peak-to-peak value adjustment time window.

FIG. 2 is a flowchart of a method for processing human biological signal data. As shown in FIG. 2, the main flow of the method is described as follows (steps S101 to S103):

Step S101: When a previous peak-to-peak value adjustment time window of the current peak-to-peak value adjustment time window ends, acquire an original peak-to-peak value of the original human biological signal within the previous peak-to-peak value adjustment time window;
in the Step S101, the original peak-to-peak value is the difference between the maximum value and the minimum value of the original human biological signal within the previous peak-to-peak value adjustment time window. Therefore, in order to be able to acquire the original peak-to-peak value, it is necessary to ensure that the peak-to-peak value adjustment time window can find a complete signal period.

Generally, the human heartbeat frequency cannot be less than 30 times per second, and a period of human biological signals such as PPG and ECG is just a heartbeat period, so the period of the original human biological signal must not be greater than 60/30 = 2 s; therefore, the duration of the peak-to-peak value adjustment time window can be set to not less than 2 s, so that a complete signal period must exist within the peak-to-peak value adjustment time window, and the maximum value and minimum value of the original human biological signal can be acquired.

Step S102: Determine a target adjustment multiple of a current peak-to-peak value adjustment time window based on the original peak-to-peak value and a preset target peak-to-peak value;
in the Step S102, the target peak-to-peak value is generally determined by the longitudinal range of the display window of the waveform display device, i.e., the target peak-to-peak value is the difference between the maximum measurement value and the minimum measurement value of the longitudinal range. Of course, the target peak-to-peak value may also be smaller than the longitudinal range of the display window, to which this embodiment is not particularly limited.

The target adjustment multiple is a ratio of the target peak-to-peak value to the original peak-to-peak value. If the original peak-to-peak value is less than the target peak-to-peak value, i.e., the target adjustment multiple is greater than 1, then the signal value within the current peak-to-peak value adjustment time window needs to be amplified; if the original peak-to-peak value is greater than the target peak-to-peak value, i.e., the target adjustment multiple is greater than 0 and less than 1, then the signal value within the current peak-to-peak value adjustment time window needs to be reduced; if the original peak-to-peak value is equal to the target peak-to-peak value, i.e. the target adjustment multiple is equal to 1, there is no need to process the signal value within the current peak-to-peak value adjustment time window.

Step S103: Determine a plurality of actual adjustment multiples of the current peak-to-peak value adjustment time window based on the target adjustment multiple, and sequentially adjust signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value.

In order to make the peak-to-peak value of the original human biological signal in the current peak-to-peak value adjustment time window gradually approach the target peak-to-peak value, a plurality of actual adjustment multiples can be determined according to the number of sampling points in the current peak-to-peak value adjustment time window, so that the plurality of actual adjustment multiples correspond to the signal values at the plurality of sampling points one by one, that is to say, when the sampling time corresponding to each sampling point in the current peak-to-peak value adjustment time window is reached, the original signal value of the sampling point is amplified or reduced according to the actual adjustment multiples corresponding to the sampling point.

In this embodiment, for the previous sampling point in the current peak-to-peak value adjustment time window, the corresponding actual adjustment multiple should not be greater than the target adjustment multiple, and the overall change trend of the actual adjustment multiple should gradually approach the target adjustment multiple, for example: the plurality of actual adjustment multiples may exhibit an increasing trend over time. Of course, it may occur that the actual adjustment times corresponding to certain sampling points fluctuate up and down.

Whether the actual adjustment multiple corresponding to the previous sampling point in the current peak-to-peak value adjustment time window reaches the target adjustment multiple or not, as long as the current peak-to-peak value adjustment time window ends, it is necessary to acquire the original peak-to-peak value according to the original human biological signal in the current peak-to-peak value adjustment time window, determine the target adjustment multiple of the next time window according to the original peak-to-peak value and the target peak-to-peak value, and continue the signal value adjustment in the next time window according to the new target adjustment multiple. That is to say, for different sampling points of the same peak-to-peak value adjustment time window, the target adjustment multiple thereof is constant; however, for the sampling points of different peak-to-peak value adjustment time windows, the target adjustment multiple thereof is generally changed, because the original peak-to-peak values may be different, but if the waveform of the original human biological signal is relatively stable, the target adjustment multiple of each peak-to-peak value adjustment time window may also be almost unchanged.

If the actual adjustment multiple reaches the target adjustment multiple when the sampling time of the previous sampling point in the current peak-to-peak adjusted time window is not reached, the actual adjustment multiple corresponding to the subsequent sampling points is set as the target adjustment multiple until the end of the current peak-to-peak value adjustment time window.

In this embodiment, the sampling time of the previous sampling point may be the end time of the current peak-to-peak value adjustment time window, or may be a time before the end of the current peak-to-peak value adjustment time window.

It should be noted that, considering the error factor, when the actual adjustment multiple belongs to the error range of the target adjustment multiple, it can be determined that the actual adjustment multiple reaches the target adjustment multiple. For example: if the target adjustment multiple is 4 and the error range thereof is $\pm 0.1$, then when the actual adjustment multiple belongs to [3.9, 4.0], it is determined that the actual adjustment multiple reaches the target adjustment multiple.

As an example to this embodiment, each actual adjustment multiple can be set by setting a single-step variation of the multiple, so that the actual adjustment multiple gradually approaches the target adjustment multiple, thereby achieving that the peak-to-peak value of the original human biological signal within the current peak-to-peak value adjustment time window gradually approaches the target peak-to-peak value. As shown in FIG. 3, the specific steps of Step S103 are as follows:

Step S1031: Determine an initial multiple single-step variation based on the target adjustment multiple and a preset initial actual adjustment multiple before acquiring a signal value of the original human biological signal at a first sampling point within the current peak-to-peak value adjustment time window;
Step S1032: Determine a second actual adjustment multiple based on the initial actual adjustment multiple and the initial multiple single-step variation, and take the second actual adjustment multiple as a current actual adjustment multiple;
Step S1033: Adjust the signal value at the first sampling point based on the current actual adjustment multiple; and
Step S1034: Determine a next multiple single-step variation based on the target adjustment multiple and the current actual adjustment multiple, determine a next actual adjustment multiple based on the current actual adjustment multiple and the next multiple single-step variation, adjust a signal value at a next sampling point based on the next actual adjustment multiple, then take the next actual adjustment multiple as a current actual adjustment multiple, and repeat the step of determining a next multiple single-step variation based on the target adjustment multiple and the current actual adjustment multiple until at least one first preset condition is satisfied;
wherein the first preset condition includes: the current actual adjustment multiple belongs to a first target interval, wherein the first target interval is determined by the target adjustment multiple; and a window end time to reach the current peak-to-peak value adjustment time window.

In this example, the first target interval is an allowable error range of the target adjustment multiple, which will not be described in detail herein.

If the current actual adjustment multiple belongs to the first target interval and the window end time of the current peak-to-peak value adjustment time window is not reached, signal values of the original human biological signal at the remaining sampling points within the current peak-to-peak value adjustment time window are adjusted based on the current actual adjustment multiple until the window end time of the current peak-to-peak value adjustment time window is reached.

Based on the above-mentioned method, except that the initial actual adjustment multiple is manually set, each actual adjustment multiple can be calculated according to the two parameters of the previous actual adjustment multiple and the single-step variation of the previous multiple jointly determined by the previous actual adjustment multiple and the target adjustment multiple, and the previous actual adjustment multiple refers to the actual adjustment multiple corresponding to the previous sampling point.

It should be noted that the initial actual adjustment multiple does not correspond to any sampling point, but is merely an initial value of the preset actual adjustment multiple, and the second actual adjustment multiple obtained based on the initial multiple single-step variation and the initial actual adjustment multiple corresponds to the first sampling point within the current peak-to-peak value adjustment time window.

The initial actual adjustment multiple is generally set as 1, and the actual adjustment multiple needs to increase from 1, so that it becomes closer to the target adjustment multiple; therefore, the calculation formula for the single-step variation of factor and the actual adjustment multiple can be respectively set as:

$$a_i=(c-b_i)/(t_1*f) \text{ Equation (1)}$$

$$b_{i+1}=b_i+a_i \text{ Equation (2)}$$

where $a_i$ represents the multiple single-step variation of the $i^{th}$ sampling point; c represents the target adjustment multiple; $b_i$ represents the actual adjustment multiple of the $i^{th}$ sampling point; $b_{i+1}$ represents the actual adjustment multiple of the $(i+1)^{th}$ sampling point; $t_1$ represents time in seconds; f represents the signal sampling rate in Hertz.

According to the above-mentioned formulas (1) to (2), the actual adjustment multiple and the value of single-step variation of multiple corresponding to each sampling point in the current peak-to-peak value adjustment time window can be obtained, see Table 1 for details.

Table 1

| Parameter / Sampling point | Target adjustment multiple c | Actual adjustment multiple $b_i$ | Multiple single-step variation $a_i$ |
|---|---|---|---|
| 0 | c | $b_0=1$ | $a_0=(c-b_0)/(t_1*f)$ |
| 1 | c | $b_1=b_0+a_0$ | $a_1=(c-b_1)/(t_1*f)$ |
| 2 | c | $b_2=b_1+a_1$ | $a_2=(c-b_2)/(t_1*f)$ |
| ...... | ...... | ...... | ...... |
| i | c | $b_i=b_{i-1}+a_{i-1}$ | $a_i=(c-b_i)/(t_1*f)$ |
| i+1 | c | $b_{i+1}=b_i+a_i$ | $a_{i+1}=(c-b_{i+1})/(t_1*f)$ |

It can be seen from Table 1 that as the value of the actual adjustment multiple increases, the value of the multiple single-step variation decreases continuously, and when the value of the multiple single-step variation decreases to or approaches 0, the value of the actual adjustment multiple increases to or approaches the target adjustment multiple.

Furthermore, as the single-step variation of the multiples becomes smaller and smaller, the change of the actual adjustment multiples becomes smaller and smaller, so the change trend of the waveform becomes more and more gentle.

When the target adjustment multiple c is greater than 1, $a_0$ is a positive number, so that $b_1 > b_0$, at this moment, the original signal value at the first sampling point is amplified, and the signal value becomes b1 times of the original value; when $b_i$ increases to be greater than c and does not belong to the first target interval, $b_i$ is a negative number, so that $b_i$, then the original signal value of the $(i+1)^{th}$ sampling point is amplified, and the signal value becomes $b_{i+1}$ times of the original value.

It can be seen that when the actual adjustment multiple exceeds the target adjustment multiple too much, the value of the actual adjustment multiple is adjusted downward by changing the multiple single-step variation to a negative number, and is adjusted downward to the first target interval one or more times, so that the actual adjustment multiple reaches the optimal value and does not change any more.

When the target adjustment multiple c is greater than 0 and less than 1, $a_0$ is a negative number, so that $0 < b_1 < b_0$, at this moment, the original signal value at the first sampling point is reduced, and the signal value becomes $b_1$ times of the original value; when $b_i$ is reduced to be smaller than c and does not belong to the first target interval, $a_i$ is a positive number, so that $b_{i+1} > b_i$, then the original signal value of the $(i+1)^{th}$ sampling point is reduced, and the signal value becomes twice the original $b_{i+1}$.

It can be seen that when the actual adjustment multiple is less than the target adjustment multiple too much, the value of the actual adjustment multiple is adjusted up by changing the multiple single-step variation to a positive number, and is adjusted up to the first target interval one or more times, so that the actual adjustment multiple reaches the optimum and does not change any more.

It should be noted that $t_1$ in the above equations (1) to (2) is generally the same as or different from the duration setting of the peak-to-peak value adjustment time window. $t_1$ represents how long it takes to adjust the original peak-peak value of the original human biological signal to the target peak-peak value; therefore, the larger $t_1$ is, the more slowly the original human biological signal changes, and the smaller $t_1$ is, the more quickly the original human biological signal changes. Therefore, in order to better suppress waveform distortion, $t_1$ is generally set to not less than 2 seconds.

The peak-to-peak value of the weak or excessively strong original human biological signal can be gradually adjusted to a suitable degree with the display area of the waveform display device by the above-mentioned waveform processing method, and FIGS. 4 and 5 successively show schematic diagrams for processing the weak or excessively strong original human biological signal by the above-mentioned waveform processing method.

For ECG signal, the thorax changes caused by human respiration, leads to offset of ECG electrodes, causing a baseline drift. However, because the baseline drift is close to the ST segment of ECG signal, if it is not properly processed, it will cause the ST segment of ECG signal distortion, resulting in misdiagnosis.

For the PPG signal, when the PPG signal is measured by Functional Near-infrared Spectroscopy (FNIRS), the detection circuit contains a DC offset, while the PPG signal itself is an AC variable. The superimposed DC offset will make the baseline of the PPG signal very high, thus making the PPG waveform in an unobvious state. In addition, when the human body performs some relatively slow activities or the ambient temperature slowly decreases, the change of human blood volume will also cause the baseline of PPG signal to change, so that the PPG waveform will present an insignificant state.

It can be seen that the original human biological signal may have baseline drift, so that the whole waveform cannot be displayed well. In order to solve the problem of baseline wandering, the signal baseline is usually adjusted to the target baseline position directly, but this will make the waveform position abrupt, resulting in waveform distortion and misdiagnosis.

In order to solve the problem of waveform distortion caused by an instantaneous offset of a waveform baseline, the embodiment of the present application is to realize a step-by-step offset of a baseline of an original human biological signal; therefore, a plurality of baseline adjustment time windows are used to perform traversal sampling on the original human biological signal, i.e., the original human biological signal is segmented according to time, and the baseline of the original physiological signal within each baseline adjustment time window is adjusted in sequence.

For two adjacent baseline adjustment time windows, the end time of the previous baseline adjustment time window is the start time of the next baseline adjustment time window. The duration of the baseline adjustment time window and the peak-to-peak value adjustment time window may be the same or different; also, the duration of each baseline adjustment time window may be the same or different, as the same for the peak-to-peak value adjustment time window.

Similar to the signal value processing method described above, the baseline offset method also discards the signal processing for the first baseline adjustment time window, which simply provides the original baseline value for the second baseline adjustment time window. However, the baseline offset processing method for the original human biological signal of any baseline adjustment time window except the first baseline adjustment time window is the same.

The waveform processing method for the original human biological signal of any baseline adjustment time window other than the first baseline adjustment time window is specifically described below, wherein any baseline adjustment time window is used as the current baseline adjustment time window.

FIG. 6 is a flowchart showing another method for processing human biological signal data. As shown in FIG. 6, the main flow of the method is described as follows (steps S201 to S203):

Step S201: When a previous baseline adjustment time window of the current baseline adjustment time window ends, acquire an original baseline value of the original human biological signal within the previous baseline adjustment time window;

In Step S201, at the end of each baseline adjustment time window, the minimum value of the original human biological signal within the baseline adjustment time window is calculated and stored in a buffer with a fixed length; in order to stabilize the data and filter out outliers, sliding median filtering and sliding mean filtering are performed on the minimum value stored in the buffer to obtain the original baseline value of the original human biological signal within the baseline adjustment time window, and the window mark is cleared to prepare for starting the next baseline adjustment time window.

Since the original baseline value is calculated as the minimum value of the original human biological signal within the previous baseline adjustment time window, it is necessary to ensure that the baseline adjustment time window can find a complete signal period. Based on the above-mentioned content regarding the value of the duration of the peak-to-peak value adjustment time window, the duration of the baseline adjustment time window can also be set to not less than 2 seconds to ensure that there must be a complete signal period within the baseline adjustment time window, and the minimum value of the original human biological signal can be obtained.

Step S202: Determine a target offset of the current baseline adjustment time window based on the original baseline value and a preset target baseline value;
in the Step S202, the target baseline value is manually set, and a default value is generally 0; however, other values may be set, to which this embodiment is not particularly limited.

The target offset is the difference between the target baseline value and the original baseline value. If the original baseline value is less than the target baseline value, i.e., the target offset is greater than 0, then the baseline position within the current baseline adjustment time window needs to be moved upwards; if the original baseline value is greater than the target baseline value, i.e., the target offset is less than 0, then the baseline position within the current baseline adjustment time window needs to be moved downwards; if the original baseline value is equal to the target baseline value, i.e. the target offset is equal to zero, then there is no need to offset the baseline position within the current baseline adjustment time window.

Step S203: Determine a plurality of actual offsets of the current baseline adjustment time window based on the target offset, and sequentially offset a baseline of the original human biological signal at a plurality of sampling points within the current baseline adjustment time window based on the plurality of actual offsets, so that the baseline of the original human biological signal within the current baseline adjustment time window gradually approaches the target baseline value.

In order to make the baseline value of the original human biological signal in the current baseline adjustment time window gradually approach the target baseline value, a plurality of actual offsets can be determined according to the number of sampling points in the current baseline adjustment time window, so that the plurality of actual offsets correspond to the signal values at the plurality of sampling points one by one, that is to say, when the sampling time corresponding to each sampling point in the current baseline adjustment time window is reached, the signal baseline of the sampling point is offset according to the actual offset corresponding to the sampling point.

For the previous sampling point in the current baseline adjustment time window, the absolute value of the corresponding actual offset shall not be greater than the absolute value of the target offset, and the overall change trend of the actual offset shall gradually approach the target offset, for example: as a function of time, the absolute values of the plurality of actual offsets may exhibit a gradually increasing trend. Of course, it may also occur that the absolute value of the actual offset corresponding to certain sampling points fluctuates upwards and downwards.

No matter whether the actual offset corresponding to the previous sampling point in the current baseline adjustment time window reaches the target offset, as long as the current baseline adjustment time window ends, it is necessary to acquire an original baseline value according to the original human biological signal in the current baseline adjustment time window, determine the target offset of the next time window according to the original baseline value and the target baseline value, and continue offsetting the baseline in the next time window according to the new target offset. That is to say, for different sampling points of the same baseline adjustment time window, the target offset thereof is constant; however, for the sampling points of different baseline adjustment time windows, the target offset is generally changed, because the original baseline value may be different, but if the waveform of the original human biological signal is relatively stable, the target offset of each baseline adjustment time window may also be almost unchanged.
If the actual offset reaches the target offset when the sampling moment of the previous sampling point in the current baseline adjustment time window is not reached, then the actual offsets corresponding to the subsequent sampling points are all set as the target offset until the end of the current baseline adjustment time window.

It should be noted that the sampling time of the previous sampling point may be the end time of the current baseline adjustment time window or a time before the end of the current baseline adjustment time window.

It should be noted that, in consideration of the error factor, when the actual offset amount belongs to the error range of the target offset amount, it can be determined that the actual offset amount reaches the target offset amount. For example: if the target offset is 10 and the error range thereof is $\pm$ 0.1, then when the actual offset belongs to [9.9, 10.1], it is determined that the actual offset reaches the target offset.

As an example to this embodiment, each actual offset may be set by setting the baseline single-step variation, so that the actual offset gradually approaches the target offset, thereby achieving that the baseline of the original human biological signal within the current peak-to-peak value adjustment time window gradually approaches the target baseline value. As shown in FIG. 7, the specific steps of Step S203 are as follows:

Step S2031: Determine an initial baseline single-step variation based on the target baseline value and a preset initial actual offset before acquiring a baseline at a first sampling point of the original human biological signal within the current baseline adjustment time window;
Step S2032: Determine a second actual offset based on the initial actual offset and the initial baseline single-step variation, and take the second actual offset as a current actual offset;
Step S2033: Offset the baseline at the first sampling point based on the current actual offset;
Step S2034: Determine a next baseline single-step variation based on the target offset and the current actual offset, determine a next actual offset based on the current actual offset and the next baseline single-step variation, offset a baseline at a next sampling point based on the next actual offset, then take the next actual offset as a current actual offset, and repeat the step of determining a next baseline single-step variation based on the target offset and the current actual offset until at least one second preset condition is satisfied;
wherein the second preset condition includes: the current actual offset belongs to a second target interval, wherein the second target interval is determined by the target offset; and reach a window end time of the current baseline adjustment time window.

In this example, the second target interval is an allowable error range of the target offset, and will not be described in detail herein.

If the current actual offset belongs to the second target interval and the window end time of the current baseline adjustment time window is not reached, the baseline of the original human biological signal at the remaining sampling points within the current baseline adjustment time window is offset based on the current actual offset until the window end time of the current baseline adjustment time window is reached.

Based on the above-mentioned method, except that the initial actual offset is manually set, each actual offset can be calculated according to the two parameters of the previous actual offset and the previous baseline single-step variation quantity jointly determined by the previous actual offset and the target offset, and the previous actual offset refers to the actual offset corresponding to the previous sampling point.

It should be noted that the initial actual offset does not correspond to any sampling point, but is merely an initial value of the preset actual offset, and the second actual offset obtained based on the initial baseline single-step variation and the initial actual offset corresponds to the first sampling point within the current baseline adjustment time window.

The initial actual offset is generally set as 0, and the absolute value of the actual offset needs to increase from 0, so that it is closer and closer to the target offset; therefore, the calculation formula for the single-step variation of the baseline and the actual offset can be respectively set as:

$$d_j=(g-e_j)/(t_2*f) \text{ equation (3)}$$

$$e_{j+1}=e_j+d_j \text{ equation (4)}$$

wherein $d_i$ represents the baseline single-step variation at the $i^{th}$ sampling point; g represents a target offset; $e_j$ represents the actual offset of the $i^{th}$ sampling point; $e_{j+1}$ represents the actual offset of the $(j+1)^{th}$ sampling point; $t_2$ represents time in seconds; and f represents the signal sampling rate in Hertz.

According to the above-mentioned equations (3) to (4), the actual offset corresponding to each sampling point in the current baseline adjustment time window and the value of the baseline single-step variation can be obtained, see Table 2 for details.

Table 2

| Parameter / Sampling point | Target offset g | Actual offset $e_j$ | Baseline single-step variation $d_j$ |
|---|---|---|---|
| 0 | g | $e_0=0$ | $d_0=(g-e_0)/(t_2*f)$ |
| 1 | g | $e_1=e_0+d_0$ | $d_1=(g-e_1)/(t_2*f)$ |
| 2 | g | $e_2=e_1+d_1$ | $d_2=(g-e_2)/(t_2*f)$ |
| …… | …… | …… | …… |
| j | g | $e_j=e_{j-1}+d_{j-1}$ | $d_j=(g-e_j)/(t_2*f)$ |
| j+1 | g | $e_{j-1}=e_j+d_j$ | $d_{j+1}=(g-e_{j+1})/(t_2*f)$ |

It can be seen from Table 2 that as the absolute value of the actual offset increases, the absolute value of the baseline single-step variation decreases, and when the value of the baseline single-step variation decreases to or approaches 0, the value of the actual offset increases to or approaches the target offset.

Furthermore, as the variation of the baseline in a single-step becomes smaller, the change of the actual offset becomes smaller, so the change trend of the waveform baseline becomes more and more gentle.

When the target offset g is greater than 0, $d_0$ is a positive number, so that $e_1 > e_0 > 0$, and at this moment, the original baseline position at the first sampling point is moved upwards by $e_1$; when $e_j$ increases to be greater than g and does not belong to the second target interval, $d_j$ is a negative number, such that $0 < e_{j+1} < e_j$, then the original baseline position of the $(j+1)^{th}$ sampling point is offset upwards by $e_{j+1}$, and the signal baseline position of the $(j+1)^{th}$ sampling point is lower than the signal baseline position of the $(j)^{th}$ sampling point.

It can be seen that when the actual offset exceeds the target offset by too much, the value of the actual offset is adjusted downward by changing the baseline single-step variation to a negative number, and is adjusted downward to the second target interval one or more times, so that the actual offset reaches the optimum and does not change any more.

When the target offset is less than 0, $d_0$ is a negative number, so that $e_1 < e_0 < 0$, and at this moment, the original baseline position at the first sampling point is moved down by $e_1$; when $e_j$ decreases to be smaller than g and does not belong to the second target interval, $d_j$ is a positive number, such that $e_j < e_{j+1} < 0$, then the original baseline position of the $(j+1)^{th}$ sampling point is offset downwards by $e_{j+1}$, and the signal baseline position of the $(j+1)^{th}$ sampling point is higher than the signal baseline position of the $(j)^{th}$ sampling point.

It can be seen that when the actual offset is too much smaller than the target offset, the value of the actual offset is adjusted up by changing the baseline single-step variation to a positive number, and is adjusted up to the second target interval one or more times, so that the actual offset reaches the optimum and does not change any more.

It should be noted that $t_2$ in the above equations (3) to (4) is generally the same as or different from the duration setting of the baseline adjustment time window. $t_2$ represents how long it takes to adjust the original baseline value of the original human biological signal to the target baseline value; therefore, the larger $t_2$ is, the slower the original human biological signal changes, and the smaller $t_2$ is, the faster the original human biological signal changes. Therefore, in order to better suppress waveform distortion, $t_2$ is generally set to not less than 2 seconds.

The baseline of the original human biological signal can be gradually adjusted to a suitable position in the display area of the waveform display device by the above waveform processing method, and FIG. 8 shows a schematic diagram of the adjustment of the baseline of the original human biological signal by the above waveform processing method.

FIG. 9 is a block diagram of an apparatus for processing human biological signal data 300 according to an embodiment of the present application. For the original human biological signal in any one of the plurality of peak-to-peak value adjustment time windows except a first peak-to-peak value adjustment time window, the any one of the peak-to-peak value adjustment time windows is taken as a current peak-to-peak value adjustment time window. As shown in FIG. 9, the apparatus for processing human biological signal data 300 mainly includes:

a first acquisition module 301 configured to, when a previous peak-to-peak value adjustment time window of the current peak-to-peak value adjustment time window ends, acquire an original peak-to-peak value of the original human biological signal within the previous peak-to-peak value adjustment time window;
a first determination module 302 configured to determine a target adjustment multiple of a current peak-to-peak value adjustment time window based on the original peak-to-peak value and a preset target peak-to-peak value; and
an adjustment module 303 configured to determine a plurality of actual adjustment multiples of the current peak-to-peak value adjustment time window based on the target adjustment multiple, and sequentially adjust signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value; wherein the plurality of actual adjustment multiples correspond to the signal values at the plurality of sampling points one by one.

As an example to this embodiment, the adjustment module 303 is specifically configured to determine a plurality of actual adjustment multiples of the current peak-to-peak value adjustment time window based on the target adjustment multiple, and sequentially adjust signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value; wherein the plurality of actual adjustment multiples correspond to the signal values at the plurality of sampling points one by one; the adjustment module is further configured to determine an initial multiple single-step variation based on the target adjustment multiple and a preset initial actual adjustment multiple before acquiring a signal value of the original human biological signal at a first sampling point within the current peak-to-peak value adjustment time window; determine a second actual adjustment multiple based on the initial actual adjustment multiple and the initial multiple single-step variation, and take the second actual adjustment multiple as a current actual adjustment multiple; adjust the signal value at the first sampling point based on the current actual adjustment multiple; determine a next multiple single-step variation based on the target adjustment multiple and the current actual adjustment multiple, determine a next actual adjustment multiple based on the current actual adjustment multiple and the next multiple single-step variation, adjust a signal value at a next sampling point based on the next actual adjustment multiple, then take the next actual adjustment multiple as the current actual adjustment multiple, and repeat the step of determining a next multiple single-step variation based on the target adjustment multiple and the current actual adjustment multiple until at least one first preset condition is satisfied; wherein the first preset condition comprises: the current actual adjustment multiple belongs to a first target interval, wherein the first target interval is determined by the target adjustment multiple; and a window end time of the current peak-to-peak value adjustment time window is reached.

In this example, the adjustment module 303 is further specifically configured to, if the current actual adjustment multiple belongs to the first target interval and the window end time of the current peak-to-peak value adjustment time window is not reached, signal values of the original human biological signal at the remaining sampling points within the current peak-to-peak value adjustment time window are adjusted based on the current actual adjustment multiple until the window end time of the current peak-to-peak value adjustment time window is reached.

As an example to this embodiment, the adjustment module 303 sets the plurality of actual adjustment times to be in an increasing trend over time.

FIG. 10 is a block diagram of an apparatus for processing human biological signal data 400 according to an embodiment of the present application. An original human biological signal is traversed with a plurality of preset baseline adjustment time windows; and for the original human biological signal in any one of the plurality of baseline adjustment time windows except a first baseline adjustment time window, the any one of the baseline adjustment time windows is taken as a current baseline adjustment time window. As shown in FIG. 10, the apparatus for processing human biological signal data 400 mainly includes:

a second acquisition module 401 configured to, when a previous baseline adjustment time window of the current baseline adjustment time window ends, acquire an original baseline value of the original human biological signal within the previous baseline adjustment time window;
a second determination module 402 configured to determine a target offset of the current baseline adjustment time window based on the original baseline value and a preset target baseline value; and
an offsetting module 403 configured to determine a plurality of actual offsets of the current baseline adjustment time window based on the target offset, and sequentially offset a baseline of the original human biological signal at a plurality of sampling points within the current baseline adjustment time window based on the plurality of actual offsets, so that the baseline of the original human biological signal within the current baseline adjustment time window gradually approaches the target baseline value.

As an example to this embodiment, an offsetting module 403 is specifically configured to determine a plurality of actual offsets of the current baseline adjustment time window based on the target offset, and sequentially offset a baseline of the original human biological signal at a plurality of sampling points within the current baseline adjustment time window based on the plurality of actual offsets, so that the baseline of the original human biological signal within the current baseline adjustment time window gradually approaches the target baseline value; wherein the plurality of actual offsets correspond to a baseline at the plurality of sampling points one by one; determine an initial baseline single-step variation based on the target baseline value and a preset initial actual offset before acquiring the baseline of the original human biological signal at a first sampling point within the current baseline adjustment time window; determine a second actual offset based on the initial actual offset and the initial baseline single-step variation, and take the second actual offset as a current actual offset; offset the baseline at the first sampling point based on the current actual offset; determine a next baseline single-step variation based on the target offset and the current actual offset, determine a next actual offset based on the current actual offset and the next baseline single-step variation, offset the baseline at the next sampling point based on the next actual offset, then take the next actual offset as the current actual offset, and repeat the step of determining a next baseline single-step variation based on the target offset and the current actual offset until at least one second preset condition is satisfied; wherein the second preset condition comprises: the current actual offset belongs to a second target interval, wherein the second target interval is determined by the target offset; and a window end time of the current baseline adjustment time window is reached.

As an example to this embodiment, the offsetting module 403 is further specifically configured to, if the current actual offset belongs to the second target interval and the window end time of the current baseline adjustment time window is not reached, offset the baseline of the original human biological signal at the remaining sampling points within the current baseline adjustment time window based on the current actual offset until the window end time of the current baseline adjustment time window is reached.

Each functional module in the embodiments of the present application can be integrated together to form an independent unit, such as being integrated into a processing unit, or each module can be physically present separately, or two or more modules can be integrated to form an independent unit. The above-mentioned integrated units may be implemented in the form of hardware or in the form of software functional units. If implemented in the form of a software function module and sold or used as a stand-alone product, the function may be stored in a computer-readable storage medium. Based on such an understanding, the technical solution of the present application, either in itself or in part contributing to the prior art, may be embodied in the form of a software product stored in a storage medium including instructions to cause an electronic device (which may be a personal computer, server or network device, etc.) or processor to perform all or part of the steps of the methods of the various embodiments of the present application. The storage medium includes: various media may store the program code, such as a U-disk, removable hard disk, read-only memory, random access memory, magnetic or optical disk.

Various modifications and specific examples of the method provided in the embodiments of the present application are also applicable to an apparatus for processing human biological signal data provided in the embodiments. From the above-mentioned detailed description of the method for processing human biological signal data, a person skilled in the art can clearly know the implementation method for the apparatus for processing human biological signal data in the embodiments, which will not be described in detail herein for the sake of brevity.

FIG. 11 is a block diagram of an electronic device 500 according to an embodiment of the present application. As shown in FIG. 11, an electronic device 500 includes a memory 501, a processor 502 and a communication bus 503; the memory 501 and the processor 502 are connected via a communication bus 503.

The memory 501 may be used to store instructions, programs, code, sets of code, or sets of instructions. The memory 501 may include a stored program area and a stored data area, wherein the stored program area may store instructions for implementing an operating system, instructions for at least one function, instructions for implementing the method for processing human biological signal data provided in the above-mentioned embodiment, etc.; the storage data area may store data or the like involved in the method for processing human biological signal data provided in the above embodiment.

The processor 502 may include one or more processing cores. The processor 502 performs various functions and processes data of the present application by running or executing instructions, programs, code sets, or instruction sets stored in memory 501 to invoke data stored in the memory 501. The processor 502 may be at least one of an Application Specific Integrated Circuit (ASIC), a Digital Signal Processor (DSP), a Digital Signal Processing Device (DSPD), a Programmable Logic Device (PLD), a Field Programmable Gate Array (FPGA), a Central Processing Unit (CPU), a controller, a microcontroller, and a microprocessor. It will be appreciated that the electronics used to implement the functions of the processor 502 described above may be other for different devices and that the embodiments of the present application are not particularly limited.

The communication bus 503 may include a path to transfer information between the described above. The communication bus 503 may be a Peripheral Component Interconnect (PCI) bus or an Extended Industry Standard Architecture (PCI) bus or the like. The communication bus 503 may be divided into an address bus, a data bus, a control bus, etc. For ease of illustration, only one double-headed arrow is shown in FIG. 11, but does not indicate that there is only one bus or type of bus. In addition, the electronic device shown in FIG. 11 is only one example and should not impose any limitation on the functionality and scope of use of the embodiments of the present application.

Embodiments of the present application provide a computer-readable storage medium storing a computer program to be loaded by a processor and executing the method for processing human biological signal data as provided in the above embodiments.

In this embodiment, a computer-readable storage medium may be a tangible device that retains and stores instructions for use by an instruction execution device. The computer-readable storage medium may be but not limited to, an electrical storage device, a magnetic storage device, an optical storage device, an electromagnetic storage device, a semiconductor storage device, or any combination of the above. In particular, the computer-readable storage medium may be a portable computer diskette, a hard disk, a U-disk, a random access memory (RAM), a read-only memory (ROM), an erasable programmable read-only memory (EPROM or Flash memory), a SRAM, a portable compact disc read-only memory (CD-ROM), a digital versatile disc (DVD), a memory stick, a floppy disk, an optical disk, a magnetic disk, a mechanical encoding device, and any combination thereof.

The computer program in this embodiment includes program code for performing the method shown in FIG. 2, FIG. 3, FIG. 6, FIG. 7, and the program code may include instructions corresponding to performing the method steps provided by the above embodiments. The computer program may be downloaded from a computer-readable storage medium to various computing/processing devices, or from an external computer or external storage device over a network, such as the Internet, a local area network, a wide area network, and/or a wireless network. The computer program may execute entirely on the user's computer as a stand-alone software package.

In the examples provided herein, it should be understood that the disclosed systems, devices, and methods may be implemented in other ways. For example, the apparatus embodiments described above are merely illustrative, e.g. partitioning of modules or units, partitioning of only one logical function, actual implementation may have additional partitioning, e.g. multiple units or components may be combined or integrated into another system, or some features may be omitted, or not performed. In another aspect, the couplings or direct couplings or communication connections shown or discussed with respect to each other may be indirect couplings or communication connections through some interface, apparatus, or unit, and may be electrical, mechanical, or otherwise.

In addition, it is to be understood that relational terms such as first and second, and the like, are used solely to distinguish one entity or action from another entity or action without necessarily requiring or implying any actual such relationship or order between such entities or actions. Further, the terms "include", "comprise", or any other variation thereof, are intended to cover a non-exclusive inclusion, such that a process, method, article, or apparatus that includes a list of elements does not include only those elements but may include other elements not expressly listed or inherent to such process, method, article, or apparatus.

The above description is only a preferred embodiment of the present application and is not intended to limit the present application, and various modifications and changes may be made to the present application by a person skilled in the art. The scope of protection is defined by the appended claims.

## Claims

1. A processor-implemented method for processing human biological signal data, wherein an original human biological signal is traversed with a plurality of preset peak-to-peak value adjustment time windows; for an original human biological signal in any one of the plurality of peak-to-peak value adjustment time windows except a first peak-to-peak value adjustment time window, the any one of the peak-to-peak value adjustment time windows is taken as a current peak-to-peak value adjustment time window, **characterized in that**, the method comprises:

   when a previous peak-to-peak value adjustment time window of the current peak-to-peak value adjustment time window ends, acquiring an original peak-to-peak value of the original human biological signal within the previous peak-to-peak value adjustment time window, wherein, the peak-to-peak value is a difference between a maximum value and a minimum value of the original human biological signal within the peak-to-peak value adjustment time window;
   determining a target adjustment multiple of the current peak-to-peak value adjustment time window based on the original peak-to-peak value and a preset target peak-to-peak value; and
   determining a plurality of actual adjustment multiples of the current peak-to-peak value adjustment time window based on the target adjustment multiple, and sequentially adjusting signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value;
   wherein the plurality of actual adjustment multiples correspond to the signal values at the plurality of sampling points one by one.

2. The method according to claim 1, **characterized in that**, the determining a plurality of actual adjustment multiples of the current peak-to-peak value adjustment time window based on the target adjustment multiple, and sequentially adjusting signal values of the original human biological signal at a plurality of sampling points within the current peak-to-peak value adjustment time window based on the plurality of actual adjustment multiples, comprises:

   determining an initial multiple single-step variation based on the target adjustment multiple and a preset initial actual adjustment multiple before acquiring a signal value of the original human biological signal at a first sampling point within the current peak-to-peak value adjustment time window;
   determining a second actual adjustment multiple based on the initial actual adjustment multiple and the initial multiple single-step variation, and taking the second actual adjustment multiple as a current actual adjustment multiple;
   adjusting the signal value at the first sampling point based on the current actual adjustment multiple; and
   determining a next multiple single-step variation based on the target adjustment multiple and the current actual adjustment multiple, determining a next actual adjustment multiple based on the current actual adjustment multiple and the next multiple single-step variation, adjusting a signal value at a next sampling point based on the next actual adjustment multiple, then taking the next actual adjustment multiple as a current actual adjustment multiple, and repeating the step of determining a next multiple single-step variation based on the target adjustment multiple and the current actual adjustment multiple until at least one first preset condition is satisfied;
   wherein the first preset condition comprises:

      the current actual adjustment multiple belongs to a first target interval, wherein the first target interval is determined by the target adjustment multiple; and
a window end time of the current peak-to-peak value adjustment time window is reached.

3. The method according to claim 2, **characterized in that**, if the current actual adjustment multiple belongs to the first target interval and the window end time of the current peak-to-peak value adjustment time window is not reached, signal values of the original human biological signal at the remaining sampling points within the current peak-to-peak value adjustment time window are adjusted based on the current actual adjustment multiple until the window end time of the current peak-to-peak value adjustment time window is reached.

4. The method according to claim 1, **characterized in that**, the plurality of actual adjustment multiples exhibit an increasing trend over time.

5. The method according to claim 1, wherein an original human biological signal is traversed with a plurality of preset baseline adjustment time windows; and for the original human biological signal in any one of the plurality of baseline adjustment time windows except a first baseline adjustment time window, the any one of the baseline adjustment time windows is taken as a current baseline adjustment time window, and the method further comprises:

when a previous baseline adjustment time window of the current baseline adjustment time window ends, acquiring an original baseline value of the original human biological signal within the previous baseline adjustment time window;
determining a target offset of the current baseline adjustment time window based on the original baseline value and a preset target baseline value; and
determining a plurality of actual offsets of the current baseline adjustment time window based on the target offset, and sequentially offsetting a baseline of the original human biological signal at a plurality of sampling points within the current baseline adjustment time window based on the plurality of actual offsets, so that the baseline of the original human biological signal within the current baseline adjustment time window gradually approaches the target baseline value;
wherein the plurality of actual offsets correspond to the baseline at the plurality of sampling points one by one.

6. The method according to claim 5, wherein the determining a plurality of actual offsets of the current baseline adjustment time window based on the target offset, and sequentially offsetting a baseline of the original human biological signal at a plurality of sampling points within the current baseline adjustment time window based on the plurality of actual offsets, comprises:

determining an initial baseline single-step variation based on the target baseline value and a preset initial actual offset before acquiring a baseline of the original human biological signal at a first sampling point within the current baseline adjustment time window;
determining a second actual offset based on the initial actual offset and the initial baseline single-step variation, and taking the second actual offset as a current actual offset;
offsetting the baseline at the first sampling point based on the current actual offset; and
determining a next baseline single-step variation based on the target offset and the current actual offset, determining a next actual offset based on the current actual offset and the next baseline single-step variation, offsetting the baseline at a next sampling point based on the next actual offset, then taking the next actual offset as a current actual offset, and repeating the step of determining a next baseline single-step variation based on the target offset and the current actual offset until at least one second preset condition is satisfied;
wherein the second preset condition comprises:
the current actual offset belongs to a second target interval, wherein the second target interval is determined by the target offset; and
a window end time of the current baseline adjustment time window is reached.

7. The method according to claim 6, wherein if the current actual offset belongs to the second target interval and the window end time of the current baseline adjustment time window is not reached, the baseline of the original human biological signal at the remaining sampling points within the current baseline adjustment time window is offset based on the current actual offset until the window end time of the current baseline adjustment time window is reached.

8. An apparatus for processing human biological signal data (300), wherein an original human biological signal is traversed with a plurality of preset peak-to-peak value adjustment time windows; and for the original human biological signal in any one of the plurality of peak-to-peak value adjustment time windows except a first peak-to-peak value adjustment time window, the any one of the peak-to-peak value adjustment time windows is taken as a current peak-to-peak value adjustment time window, and **characterized in that**, the apparatus comprises:

a first acquisition module (301) configured to, when a previous peak-to-peak value adjustment time window of the current peak-to-peak value adjustment time window ends, acquire an original peak-to-peak value of the original human biological signal within the previous peak-to-peak value adjustment time window, wherein, the peak-to-peak value is a difference between a maximum value and a minimum value of the original human biological signal within the peak-to-peak value adjustment time window;
a first determination module (302) configured to determine a target adjustment multiple of the current peak-to-peak value adjustment time window based on the original peak-to-peak value and a preset target peak-to-peak value; and
an adjustment module (303) configured to determine a plurality of actual adjustment multiples of the current peak-to-peak value adjustment time window based on the target adjustment multiple, and sequentially adjust signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value; wherein the plurality of actual adjustment multiples correspond to the signal values at the plurality of sampling points one by one.

**9.** The apparatus according to claim 8, wherein the adjustment module (303) is further configured to determine an initial multiple single-step variation based on the target adjustment multiple and a preset initial actual adjustment multiple before acquiring a signal value of the original human biological signal at a first sampling point within the current peak-to-peak value adjustment time window; determine a second actual adjustment multiple based on the initial actual adjustment multiple and the initial multiple single-step variation, and take the second actual adjustment multiple as a current actual adjustment multiple; adjust the signal value at the first sampling point based on the current actual adjustment multiple; determine a next multiple single-step variation based on the target adjustment multiple and the current actual adjustment multiple, determine a next actual adjustment multiple based on the current actual adjustment multiple and the next multiple single-step variation, adjust a signal value at a next sampling point based on the next actual adjustment multiple, then take the next actual adjustment multiple as the current actual adjustment multiple, and repeat the step of determining a next multiple single-step variation based on the target adjustment multiple and the current actual adjustment multiple until at least one first preset condition is satisfied; wherein the first preset condition comprises: the current actual adjustment multiple belongs to a first target interval, wherein the first target interval is determined by the target adjustment multiple; and a window end time of the current peak-to-peak value adjustment time window is reached.

**10.** An electronic device (500), comprising a memory (501) and a processor (502); **characterized in that**, the memory (501) has stored thereon a computer program to be loaded by the processor (502) to perform the method according to any one of claims 1 to 7.

**11.** A computer-readable storage medium, **characterized in that**, the computer-readable storage medium has stored thereon a computer program to be loaded by a processor (502) to perform the method according to any one of claims 1 to 7.

## Patentansprüche

**1.** Prozessorimplementiertes Verfahren zur Verarbeitung menschlicher biologischer Signaldaten, bei dem ein ursprüngliches menschliches biologisches Signal mit einer Mehrzahl von voreingestellten Zeitfenstern zur Anpassung des Spitze-Spitze-Werts durchlaufen wird; für ein ursprüngliches menschliches biologisches Signal in einem beliebigen der mehreren Zeitfenster zur Anpassung des Spitze-Spitze-Werts, mit Ausnahme eines ersten Zeitfensters zur Anpassung des Spitze-Spitze-Werts, wird das beliebige der Zeitfenster zur Anpassung des Spitze-Spitze-Werts als aktuelles Zeitfenster zur Anpassung des Spitze-Spitze-Werts herangezogen, **dadurch gekennzeichnet, dass** das Verfahren umfasst:

wenn ein vorheriges Zeitfenster zur Anpassung des Spitze-Spitze-Werts des aktuellen Zeitfensters zur Anpassung des Spitze-Spitze-Werts endet, Erfassen eines ursprünglichen Spitze-Spitze-Werts des ursprünglichen menschlichen biologischen Signals innerhalb des vorherigen Zeitfensters zur Anpassung des Spitze-Spitze-Werts, wobei der Spitze-Spitze-Wert eine Differenz zwischen einem Maximalwert und einem Minimalwert des ursprünglichen menschlichen biologischen Signals innerhalb des Zeitfensters zur Anpassung des Spitze-Spitze-Werts ist;

Bestimmen eines Soll-Anpassungsmultiplikators für das aktuelle Zeitfenster zur Anpassung des Spitze-Spitze-Werts auf der Grundlage des ursprünglichen Spitze-Spitze-Werts und eines voreingestellten Soll-Spitze-Spitze-Werts; und

Bestimmen einer Mehrzahl von tatsächlichen Anpassungsfaktoren für das aktuelle Zeitfenster zur Anpassung des Spitze-Spitze-Werts auf der Grundlage des Zielanpassungsfaktors und sequentielles Anpassen von Signalwerten des ursprünglichen menschlichen biologischen Signals an einer Mehrzahl von Abtastpunkten innerhalb des aktuellen Zeitfensters zur Anpassung des Spitze-Spitze-Werts auf der Grundlage der Mehrzahl von tatsächlichen Anpassungsfaktoren, so dass sich ein Spitze-Spitze-Wert des ursprünglichen menschlichen biologischen Signals innerhalb des aktuellen Zeitfensters zur Anpassung des Spitze-Spitze-Werts allmählich dem Ziel-Spitze-Spitze-Wert annähert;

wobei die mehreren tatsächlichen Anpassungsfaktoren nacheinander den Signalwerten an den mehreren Abtastpunkten entsprechen.

2. Verfahren nach Anspruch 1, **dadurch gekennzeichnet, dass** das Bestimmen einer Mehrzahl von tatsächlichen Anpassungsmultiplikatoren des aktuellen Zeitfensters zur Anpassung des Spitze-Spitze-Werts auf der Grundlage des Zielanpassungsmultiplikators und das sequenzielle Anpassen von Signalwerten des ursprünglichen menschlichen biologischen Signals an einer Mehrzahl von Abtastpunkten innerhalb des aktuellen Zeitfensters zur Anpassung des Spitze-Spitze-Werts auf der Grundlage der Mehrzahl von tatsächlichen Anpassungs -Multiplikatoren umfasst:

   Bestimmen einer anfänglichen Ein-Schritt-Variation des Multiplikators auf der Grundlage des Ziel-Anpassungsmultiplikators und eines voreingestellten anfänglichen tatsächlichen Anpassungsmultiplikators, bevor ein Signalwert des ursprünglichen menschlichen biologischen Signals an einem ersten Abtastpunkt innerhalb des aktuellen Spitze-Spitzewert-Anpassungszeitfensters erfasst wird;

   Bestimmen eines zweiten tatsächlichen Anpassungsmultiplikators auf der Grundlage des anfänglichen tatsächlichen Anpassungsmultiplikators und der anfänglichen mehrfachen Einzelschrittabweichung und Anwenden des zweiten tatsächlichen Anpassungsmultiplikators als aktuellen tatsächlichen Anpassungsmultiplikator;

   Anpassen des Signalwerts am ersten Abtastpunkt auf der Grundlage des aktuellen tatsächlichen Anpassungsmultiplikators; und

   Bestimmen einer nächsten mehrfachen Einzelschrittabweichung auf der Grundlage des Zielanpassungsmultiplikators und des aktuellen tatsächlichen Anpassungsmultiplikators, Bestimmen eines nächsten tatsächlichen Anpassungsmultiplikators auf der Grundlage des aktuellen tatsächlichen Anpassungsmultiplikators und der nächsten mehrfachen Einzelschrittabweichung, Anpassen eines Signalwerts an einem nächsten Abtastpunkt auf der Grundlage des nächsten tatsächlichen Anpassungsmultiplikators, anschließendes Übernehmen des nächsten tatsächlichen Anpassungsmultiplikators als aktuellen tatsächlichen Anpassungsmultiplikator, und Wiederholen des Schritts des Bestimmens einer nächsten mehrfachen Einzelschrittabweichung auf der Grundlage des Zielanpassungsmultiplikators und des aktuellen tatsächlichen Anpassungsmultiplikators, bis mindestens eine erste voreingestellte Bedingung erfüllt ist;

   wobei die erste voreingestellte Bedingung umfasst:

      der aktuelle tatsächliche Anpassungsmultiplikator gehört zu einem ersten Zielintervall, wobei das erste Zielintervall durch den Zielanpassungsmultiplikator bestimmt wird; und

      eine Fensterendzeit des aktuellen Spitze-Spitze-Wert-Anpassungszeitfensters ist erreicht.

3. Verfahren nach Anspruch 2, **dadurch gekennzeichnet, dass**, wenn das aktuelle tatsächliche Anpassungsmultiplikationsfaktor zum ersten Zielintervall gehört und die Fensterendzeit des aktuellen Spitze-Spitze-Wert-Anpassungszeitfensters nicht erreicht ist, Signalwerte des ursprünglichen menschlichen biologischen Signals an den verbleibenden Abtastpunkten innerhalb des aktuellen Spitze-Spitze-Wert-Anpassungszeitfensters auf der Grundlage des aktuellen tatsächlichen Anpassungsmultiplikationsfaktors angepasst werden, bis die Fensterendzeit des aktuellen Spitze-Spitze-Wert-Anpassungszeitfensters erreicht ist.

4. Verfahren nach Anspruch 1, **dadurch gekennzeichnet, dass** die Mehrzahl von tatsächlichen Anpassungsfaktoren im Laufe der Zeit einen steigenden Trend aufweist.

5. Verfahren nach Anspruch 1, wobei ein ursprüngliches menschliches biologisches Signal mit einer Mehrzahl von voreingestellten Basislinien-Anpassungszeitfenstern durchlaufen wird; und für das ursprüngliche menschliche biologische Signal in einem beliebigen der mehreren Basislinien-Anpassungszeitfenstern mit Ausnahme eines ersten Basislinien-Anpassungszeitfensters wird das beliebige Basislinien-Anpassungszeitfenster als aktuelles Basislinien-Anpassungszeitfenster herangezogen, und das Verfahren umfasst ferner:

wenn ein vorheriges Basislinienanpassungs-Zeitfenster des aktuellen Basislinienanpassungs-Zeitfensters endet, Erfassen eines ursprünglichen Basislinienwerts des ursprünglichen menschlichen biologischen Signals innerhalb des vorherigen Basislinienanpassungs-Zeitfensters;
Bestimmen eines Ziel-Offsets des aktuellen Basislinienanpassungs-Zeitfensters auf der Grundlage des ursprünglichen Basislinienwerts und eines voreingestellten Ziel-Basislinienwerts; und
Bestimmen einer Mehrzahl von tatsächlichen Offsets des aktuellen Basislinienanpassungs-Zeitfensters auf der Grundlage des Ziel-Offsets und sequentielles Versetzen einer Basislinie des ursprünglichen menschlichen biologischen Signals an einer Mehrzahl von Abtastpunkten innerhalb des aktuellen Basislinienanpassungs-Zeitfensters auf der Grundlage der Mehrzahl von tatsächlichen Offsets, so dass sich die Basislinie des ursprünglichen menschlichen biologischen Signals innerhalb des aktuellen Basislinienanpassungs-Zeitfensters allmählich dem Ziel-Basislinienwert annähert;
wobei die mehreren tatsächlichen Offsets nacheinander der Basislinie an den mehreren Abtastpunkten entsprechen.

**6.** Verfahren nach Anspruch 5, wobei das Bestimmen einer Mehrzahl von tatsächlichen Offsets des aktuellen Basislinienanpassungs-Zeitfensters auf der Grundlage des Ziel-Offsets und das sequenzielle Versetzen einer Basislinie des ursprünglichen menschlichen biologischen Signals an einer Mehrzahl von Abtastpunkten innerhalb des aktuellen Basislinienanpassungs-Zeitfensters auf der Grundlage der Mehrzahl von tatsächlichen Offsets umfasst:

Bestimmen einer anfänglichen Basislinien-Einzelschrittabweichung auf der Grundlage des Ziel-Basislinienwerts und eines voreingestellten anfänglichen tatsächlichen Offsets, bevor eine Basislinie des ursprünglichen menschlichen biologischen Signals an einem ersten Abtastpunkt innerhalb des aktuellen Basislinienanpassungs-Zeitfensters erfasst wird;
Bestimmen eines zweiten tatsächlichen Offsets auf der Grundlage des anfänglichen tatsächlichen Offsets und der anfänglichen Basislinien-Einzelschrittabweichung und Verwenden des zweiten tatsächlichen Offsets als aktuellen tatsächlichen Offset;
Versetzen der Basislinie am ersten Abtastpunkt auf der Grundlage des aktuellen tatsächlichen Offsets; und
Bestimmen einer nächsten Basislinien-Einzelschrittabweichung auf der Grundlage des Ziel-Offsets und des aktuellen tatsächlichen Offsets, Bestimmen eines nächsten tatsächlichen Offsets auf der Grundlage des aktuellen tatsächlichen Offsets und der nächsten Basislinien-Einzelschrittabweichung, Verschieben der Basislinie an einem nächsten Abtastpunkt auf der Grundlage des nächsten tatsächlichen Offsets, anschließendes Übernehmen des nächsten tatsächlichen Offsets als aktuellen tatsächlichen Offset und Wiederholen des Schritts des Bestimmens einer nächsten Basislinien-Einzelschrittabweichung auf der Grundlage des Ziel-Offsets und des aktuellen tatsächlichen Offsets, bis mindestens eine zweite voreingestellte Bedingung erfüllt ist;
wobei die zweite voreingestellte Bedingung umfasst:
der aktuelle Ist-Versatz gehört zu einem zweiten Zielintervall, wobei das zweite Zielintervall durch den Soll-Versatz bestimmt wird; und
eine Fensterendzeit des aktuellen Zeitfensters zur Basislinienanpassung ist erreicht.

**7.** Verfahren nach Anspruch 6, wobei, wenn der aktuelle tatsächliche Offset zu dem zweiten Zielintervall gehört und die Fensterendzeit des aktuellen Basislinienanpassungs-Zeitfensters nicht erreicht ist, die Basislinie des ursprünglichen menschlichen biologischen Signals an den verbleibenden Abtastpunkten innerhalb des aktuellen Basislinienanpassungs-Zeitfensters auf der Grundlage des aktuellen tatsächlichen Offsets versetzt wird, bis die Fensterendzeit des aktuellen Basislinienanpassungs-Zeitfensters erreicht ist.

**8.** Vorrichtung zur Verarbeitung von menschlichen biologischen Signaldaten (300), wobei ein ursprüngliches menschliches biologisches Signal mit einer Mehrzahl von voreingestellten Zeitfenstern zur Anpassung des Spitze-Spitze-Werts durchlaufen wird; und für das ursprüngliche menschliche biologische Signal in einem beliebigen der mehreren Zeitfenster zur Anpassung des Spitze-Spitze-Werts, mit Ausnahme eines ersten Zeitfensters zur Anpassung des Spitze-Spitze-Werts, das beliebige der Zeitfenster zur Anpassung des Spitze-Spitze-Werts als aktuelles Zeitfenster zur Anpassung des Spitze-Spitze-Werts herangezogen wird, und **dadurch gekennzeichnet, dass** die Vorrichtung umfasst:

ein erstes Erfassungsmodul (301), das so konfiguriert ist, dass es, wenn ein vorheriges Zeitfenster zur Anpassung des Spitze-Spitze-Werts des aktuellen Zeitfensters zur Anpassung des Spitze-Spitze-Werts endet, einen ursprünglichen Spitze-Spitze-Wert des ursprünglichen menschlichen biologischen Signals innerhalb des vorherigen Zeitfensters zur Anpassung des Spitze-Spitze-Werts erfasst, wobei der Spitze-Spitze-Wert eine Differenz zwischen einem Maximalwert und einem Minimalwert des ursprünglichen menschlichen biologischen Signals innerhalb des Zeitfensters zur Anpassung des Spitze-Spitze-Werts ist;

ein erstes Bestimmungsmodul (302), das so konfiguriert ist, dass es auf der Grundlage des ursprünglichen Spitze-Spitze-Werts und eines voreingestellten Soll-Spitze-Spitze-Werts einen Soll-Anpassungsfaktor für das aktuelle Anpassungszeitfenster für den Spitze-Spitze-Wert bestimmt; und

ein Anpassungsmodul (303), das so konfiguriert ist, dass es eine Mehrzahl von tatsächlichen Anpassungsfaktoren für das aktuelle Spitze-Spitze-Wert-Anpassungszeitfenster auf der Grundlage des Zielanpassungsfaktors bestimmt und Signalwerte des ursprünglichen menschlichen biologischen Signals an einer Mehrzahl von Abtastpunkten innerhalb des aktuellen Spitze-Spitze-Wert-Anpassungszeitfensters auf der Grundlage der Mehrzahl von tatsächlichen Anpassungsfaktoren sequenziell anpasst, so dass sich ein Spitze-Spitze-Wert des ursprünglichen menschlichen biologischen Signals innerhalb des aktuellen Spitze-Spitze-Wert-Anpassungszeitfensters allmählich dem Ziel-Spitze-Spitze-Wert annähert; wobei die Mehrzahl von tatsächlichen Anpassungsmultiplikatoren nacheinander den Signalwerten an den Mehrzahl von Abtastpunkten entspricht.

**9.** Vorrichtung nach Anspruch 8, wobei das Anpassungsmodul (303) ferner so konfiguriert ist, dass es eine anfängliche einstufige Variation des Multiplikators auf der Grundlage des Ziel-Anpassungsmultiplikators und eines voreingestellten anfänglichen tatsächlichen Anpassungsmultiplikators bestimmt, bevor ein Signalwert des ursprünglichen menschlichen biologischen Signals an einem ersten Abtastpunkt innerhalb des aktuellen Zeitfensters zur Anpassung des Spitze-Spitze-Werts erfasst wird; einen zweiten tatsächlichen Anpassungsmultiplikationsfaktor auf der Grundlage des anfänglichen tatsächlichen Anpassungsmultiplikationsfaktors und der anfänglichen mehrfachen Ein-Schritt-Variation zu bestimmen und den zweiten tatsächlichen Anpassungsmultiplikationsfaktor als aktuellen tatsächlichen Anpassungsmultiplikationsfaktor zu verwenden; den Signalwert am ersten Abtastpunkt auf der Grundlage des aktuellen tatsächlichen Anpassungsmultiplikators anzupassen; eine nächste mehrfache Einzelschrittabweichung auf der Grundlage des Zielanpassungsmultiplikators und des aktuellen tatsächlichen Anpassungsmultiplikators zu bestimmen, einen nächsten tatsächlichen Anpassungsmultiplikator auf der Grundlage des aktuellen tatsächlichen Anpassungsmultiplikators und der nächsten mehrfachen Einzelschrittabweichung zu bestimmen, einen Signalwert an einem nächsten Abtastpunkt auf der Grundlage des nächsten tatsächlichen Anpassungsmultiplikators anzupassen, dann den nächsten tatsächlichen Anpassungsmultiplikator als aktuellen tatsächlichen Anpassungsmultiplikator zu übernehmen, und Wiederholen des Schritts des Bestimmens einer nächsten mehrfachen Einzelschrittabweichung auf der Grundlage des Zielanpassungsmultiplikators und des aktuellen tatsächlichen Anpassungs s, bis mindestens eine erste voreingestellte Bedingung erfüllt ist; wobei die erste voreingestellte Bedingung umfasst: der aktuelle tatsächliche Anpassungsmultiplikator gehört zu einem ersten Zielintervall, wobei das erste Zielintervall durch den Zielanpassungsmultiplikator bestimmt wird; und eine Fensterendzeit des aktuellen Spitze-Spitze-Wert-Anpassungszeitfensters erreicht ist.

**10.** Elektronisches Gerät (500), umfassend einen Speicher (501) und einen Prozessor (502); **dadurch gekennzeichnet, dass** auf dem Speicher (501) ein Computerprogramm gespeichert ist, das vom Prozessor (502) geladen wird, um das Verfahren gemäß einem der Ansprüche 1 bis 7 auszuführen.

**11.** Computerlesbares Speichermedium, **dadurch gekennzeichnet, dass** auf dem computerlesbaren Speichermedium ein Computerprogramm gespeichert ist, das von einem Prozessor (502) geladen wird, um das Verfahren gemäß einem der Ansprüche 1 bis 7 auszuführen.

## Revendications

**1.** Procédé mis en œuvre par processeur pour traiter des données de signal biologique humain, en ce qu'un signal biologique humain d'origine est parcouru avec une pluralité de fenêtres de temps d'ajustement de valeur de crête à crête prédéfinies ; pour un signal biologique humain d'origine dans l'une quelconque de la pluralité de fenêtres de temps d'ajustement de valeur de crête à crête, à l'exception d'une première fenêtre de temps d'ajustement de valeur de crête à crête, ladite fenêtre de temps d'ajustement de valeur de crête à crête est considérée comme une fenêtre de temps d'ajustement de valeur de crête à crête actuelle, **caractérisé en ce que** le procédé comprend :

lorsqu'une fenêtre de temps d'ajustement de valeur de crête à crête précédente des fenêtres de temps d'ajustement de valeur de crête à crête actuelles se termine, la saisie d'une valeur de crête à crête d'origine du signal biologique humain d'origine à l'intérieur de la fenêtre de temps d'ajustement de valeur de crête à crête précédente, **en ce que** la valeur de crête à crête est une différence entre une valeur maximale et une valeur minimale du signal biologique humain d'origine à l'intérieur de la fenêtre de temps d'ajustement de valeur de crête à crête ;
la détermination d'un multiple d'ajustement cible de la fenêtre de temps d'ajustement de valeur de crête à crête actuelle sur la base de la valeur de crête à crête d'origine et d'une valeur de crête à crête cible prédéfinie ; et
la détermination d'une pluralité de multiples d'ajustement réels de la fenêtre de temps d'ajustement de valeur de crête à crête actuelle sur la base du multiple d'ajustement cible et, séquentiellement, l'ajustement de valeurs de signal du signal biologique humain d'origine à une pluralité de points d'échantillonnage à l'intérieur de la fenêtre de temps d'ajustement de valeur de crête à crête actuelle sur la base de la pluralité de multiples d'ajustement réels, de sorte qu'une valeur de crête à crête du signal biologique humain d'origine à l'intérieur de la fenêtre de temps d'ajustement de valeur de crête à crête actuelle se rapproche progressivement de la valeur de crête à crête cible ;
**en ce que** la pluralité de multiples d'ajustement réels correspondent aux valeurs de signal au niveau de la pluralité de points d'échantillonnage un par un.

2. Procédé selon la revendication 1, **caractérisé en ce que** la détermination d'une pluralité de multiples d'ajustement réels de la fenêtre de temps d'ajustement de valeur de crête à crête actuelle sur la base du multiple d'ajustement cible et, séquentiellement, l'ajustement de valeurs de signal du signal biologique humain d'origine à une pluralité de points d'échantillonnage à l'intérieur de la fenêtre de temps d'ajustement de valeur de crête à crête actuelle sur la base de la pluralité de multiples d'ajustement réels, comprend :

la détermination d'une variation en une seule étape du multiple initiale sur la base du multiple d'ajustement cible et d'un multiple d'ajustement réel initial prédéfini avant l'acquisition d'une valeur de signal du signal biologique humain d'origine à un premier point d'échantillonnage à l'intérieur de la fenêtre de temps d'ajustement de valeur de crête à crête actuelle ;
la détermination d'un deuxième multiple d'ajustement réel sur la base du multiple d'ajustement réel initial et de la variation en une seule étape du multiple initiale, et la prise en compte du deuxième multiple d'ajustement réel comme multiple d'ajustement réel actuel ;
l'ajustement de la valeur de signal au premier point d'échantillonnage sur la base du multiple d'ajustement réel actuel ; et
la détermination d'une variation en une seule étape de multiple suivante sur la base du multiple d'ajustement cible et du multiple d'ajustement réel actuel, la détermination d'un multiple d'ajustement réel suivant sur la base du multiple d'ajustement réel actuel et de la variation en une seule étape de multiple suivante, l'ajustement d'une valeur de signal au point d'échantillonnage suivant sur la base du multiple d'ajustement réel suivant, puis la prise en compte du multiple d'ajustement réel suivant comme multiple d'ajustement réel actuel, et la répétition de l'étape consistant à déterminer une variation en une seule étape de multiple suivante sur la base du multiple d'ajustement cible et du multiple d'ajustement réel actuel jusqu'à ce qu'au moins une première condition prédéfinie soit satisfaite ;
**en ce que** la première condition prédéfinie consiste à ce que :

le multiple d'ajustement réel actuel appartienne à un premier intervalle cible, **en ce que** le premier intervalle cible est déterminé par le multiple d'ajustement cible ; et
une fin de fenêtre de la fenêtre de temps d'ajustement de valeur de crête à crête actuelle soit atteinte.

3. Procédé selon la revendication 2, **caractérisé en ce que**, si le multiple d'ajustement réel actuel appartient au premier intervalle cible et si la fin de fenêtre de la fenêtre de temps d'ajustement de valeur de crête à crête actuelle n'est pas atteinte, des valeurs de signal du signal biologique humain d'origine aux points d'échantillonnage restants à l'intérieur de la fenêtre de temps d'ajustement de valeur de crête à crête actuelle sont ajustées sur la base du multiple d'ajustement réel actuel jusqu'à ce que la fin de fenêtre de la fenêtre de temps d'ajustement de valeur de crête à crête actuelle soit atteinte.

4. Procédé selon la revendication 1, **caractérisé en ce que** la pluralité de multiples d'ajustement réels ont tendance à augmenter au fil du temps.

5. Procédé selon la revendication 1, en ce qu'un signal biologique humain d'origine est parcouru avec une pluralité de fenêtres de temps d'ajustement de ligne de base prédéfinies ; et pour le signal biologique humain d'origine dans l'une quelconque de la pluralité de fenêtres de temps d'ajustement de ligne de base, à l'exception d'une première fenêtre de temps d'ajustement de ligne de base, l'une quelconque des fenêtres de temps d'ajustement de ligne de base est considérée comme fenêtre de temps d'ajustement de ligne de base actuelle, et le procédé comprend en outre :

lorsqu'une fenêtre de temps d'ajustement de ligne de base précédente de la fenêtre de temps d'ajustement de ligne de base actuelle se termine, la saisie d'une valeur de ligne de base d'origine du signal biologique humain d'origine à l'intérieur de la fenêtre de temps d'ajustement de ligne de base précédente ;
la détermination d'un décalage cible de la fenêtre de temps d'ajustement de ligne de base actuelle sur la base de la valeur de ligne de base d'origine et d'une valeur de ligne de base cible prédéfinie ; et
la détermination d'une pluralité de décalages réels de la fenêtre de temps d'ajustement de ligne de base actuelle sur la base du décalage cible et, séquentiellement, le décalage d'une ligne de base du signal biologique humain d'origine à une pluralité de points d'échantillonnage à l'intérieur de la fenêtre de temps d'ajustement de ligne de base actuelle sur la base de la pluralité de décalages réels, de sorte que la ligne de base du signal biologique humain d'origine à l'intérieur de la fenêtre de temps d'ajustement de ligne de base actuelle se rapproche progressivement de la valeur de ligne de base cible ;
en ce que la pluralité de décalages réels correspondent à la ligne de base au niveau de la pluralité de points d'échantillonnage un par un.

**6.** Procédé selon la revendication 5, en ce que la détermination d'une pluralité de décalages réels de la fenêtre de temps d'ajustement de ligne de base actuelle sur la base du décalage cible et, séquentiellement, le décalage d'une ligne de base du signal biologique humain d'origine à une pluralité de points d'échantillonnage à l'intérieur de la fenêtre de temps d'ajustement de ligne de base actuelle sur la base de la pluralité de décalages réels, comprend :

la détermination d'une variation en une seule étape de ligne de base initiale sur la base de la valeur de ligne de base cible et d'un décalage réel initial prédéfini avant l'acquisition d'une ligne de base du signal biologique humain d'origine à un premier point d'échantillonnage à l'intérieur de la fenêtre de temps d'ajustement de ligne de base actuelle ;
la détermination d'un deuxième décalage réel sur la base du décalage réel initial et de la variation en une seule étape de ligne de base initiale, et la prise en compte du deuxième décalage réel comme décalage réel actuel ;
le décalage de la ligne de base au premier point d'échantillonnage sur la base du décalage réel actuel ; et
la détermination d'une variation en une seule étape de ligne de base suivante sur la base du décalage cible et du décalage réel actuel, la détermination d'un décalage réel suivant sur la base du décalage réel actuel et de la variation en une seule étape de ligne de base suivante, le décalage de la ligne de base à un point d'échantillonnage suivant sur la base du décalage réel suivant, puis la prise en compte du décalage réel suivant comme décalage réel actuel, et la répétition de l'étape consistant à déterminer une variation en une seule étape de ligne de base suivante sur la base du décalage cible et du décalage réel actuel jusqu'à ce qu'au moins une deuxième condition prédéfinie soit satisfaite ;
en ce que la deuxième condition prédéfinie consiste à ce que :

le décalage réel actuel appartienne à un deuxième intervalle cible, en ce que le deuxième intervalle cible est déterminé par le décalage cible ; et
la fin de fenêtre de la fenêtre de temps d'ajustement de ligne de base actuelle soit atteinte.

**7.** Procédé selon la revendication 6 en ce que, si le décalage réel actuel appartient au deuxième intervalle cible et la fin de fenêtre de la fenêtre de temps d'ajustement de ligne de base actuelle n'est pas atteint, la ligne de base du signal biologique humain d'origine aux points d'échantillonnage restants à l'intérieur de la fenêtre de temps d'ajustement de ligne de base actuelle est décalée sur la base du décalage réel actuel jusqu'à ce que la fin de fenêtre de la fenêtre de temps d'ajustement de ligne de base actuelle soit atteinte.

**8.** Appareil de traitement de données de signal biologique humain (300), en ce qu'un signal biologique humain d'origine est parcouru avec une pluralité de fenêtres de temps d'ajustement de valeur de crête à crête prédéfinies ; et pour le signal biologique humain d'origine dans l'une quelconque de la pluralité de fenêtres de temps d'ajustement de valeur de crête à crête, à l'exception d'une première fenêtre de temps d'ajustement de valeur de crête à crête, l'une quelconque des fenêtres de temps d'ajustement de valeur de crête à crête est considérée comme une fenêtre de temps d'ajustement de valeur de crête à crête actuelle, et **caractérisé en ce que** l'appareil comprend :

un premier module d'acquisition (301) configuré pour, lorsqu'une fenêtre de temps d'ajustement de valeur de crête à crête précédente de la fenêtre de temps d'ajustement de valeur de crête à crête actuelle se termine, la saisie d'une valeur de crête à crête d'origine du signal biologique humain d'origine à l'intérieur de la fenêtre de temps d'ajustement de valeur de crête à crête précédente, **en ce que** la valeur de crête à crête est une différence entre une valeur maximale et une valeur minimale du signal biologique humain d'origine à l'intérieur de la fenêtre de temps d'ajustement de valeur de crête à crête ;
un premier module de détermination (302) configuré pour déterminer un multiple d'ajustement cible de la fenêtre de temps d'ajustement de valeur de crête à crête actuelle sur la base de la valeur de crête à crête d'origine et d'une valeur de crête à crête cible prédéfinie ; et
un module d'ajustement (303) configuré pour déterminer une pluralité de multiples d'ajustement réels de la fenêtre de temps d'ajustement de valeur de crête à crête actuelle sur la base du multiple d'ajustement cible et, séquentiellement, pour ajuster des valeurs de signal du signal biologique humain d'origine à une pluralité de points d'échantillonnage à l'intérieur de la fenêtre de temps d'ajustement de valeur de crête à crête actuelle sur la base de la pluralité des multiples d'ajustement réels, de sorte qu'une valeur de crête à crête du signal biologique humain d'origine à l'intérieur de la fenêtre de temps d'ajustement de valeur de crête à crête actuelle se rapproche progressivement de la valeur de crête à crête cible ;
**en ce que** la pluralité de multiples d'ajustement réels correspondent aux valeurs de signal au niveau de la pluralité de points d'échantillonnage un par un.

**9.** Appareil selon la revendication 8, en ce que le module d'ajustement (303) est en outre configuré pour déterminer une variation en une seule étape de multiple initiale sur la base du multiple d'ajustement cible et d'un multiple d'ajustement initial prédéfini avant l'acquisition d'une valeur de signal du signal biologique humain d'origine à un premier point d'échantillonnage à l'intérieur de la fenêtre de temps d'ajustement de valeur de crête à crête actuelle ; pour déterminer un deuxième multiple d'ajustement réel sur la base du multiple d'ajustement réel initial et de la variation en une seule étape de multiple initiale, et pour considérer le deuxième multiple d'ajustement réel comme multiple d'ajustement réel actuel ; pour ajuster la valeur de signal au premier point d'échantillonnage sur la base du multiple d'ajustement réel actuel ; pour déterminer une variation en une seule étape de multiple suivante sur la base du multiple d'ajustement cible et du multiple d'ajustement réel actuel, pour déterminer un multiple d'ajustement réel suivant sur la base du multiple d'ajustement réel actuel et de la variation en une seule étape de multiple suivante, pour ajuster une valeur de signal à un point d'échantillonnage suivant sur la base du multiple d'ajustement réel suivant, puis pour considérer le multiple d'ajustement réel suivant comme multiple d'ajustement réel actuel, et pour répéter l'étape consistant à déterminer une variation en une seule étape de multiple suivante sur la base du multiple d'ajustement cible et du multiple d'ajustement réel actuel jusqu'à ce qu'au moins une première condition prédéfinie soit satisfaite ; en ce que la première condition prédéfinie consiste à ce que : le multiple d'ajustement réel actuel appartienne à un premier intervalle cible, en ce que le premier intervalle cible est déterminé par le multiple d'ajustement cible ; et la fin de fenêtre de la fenêtre de temps d'ajustement de valeur de crête à crête actuelle soit atteinte.

**10.** Dispositif électronique (500) comprenant une mémoire (501) et un processeur (502) ; **caractérisé en ce que** la mémoire (501) y a stocké un programme informatique destiné à être chargé par le processeur (502) afin de mettre en œuvre le procédé selon l'une des revendications 1 à 7.

**11.** Support de stockage lisible par ordinateur, **caractérisé en ce que** le support de stockage lisible par ordinateur y a stocké un programme informatique destiné à être chargé par un processeur (502) afin de mettre en œuvre le procédé selon l'une des revendications 1 à 7.

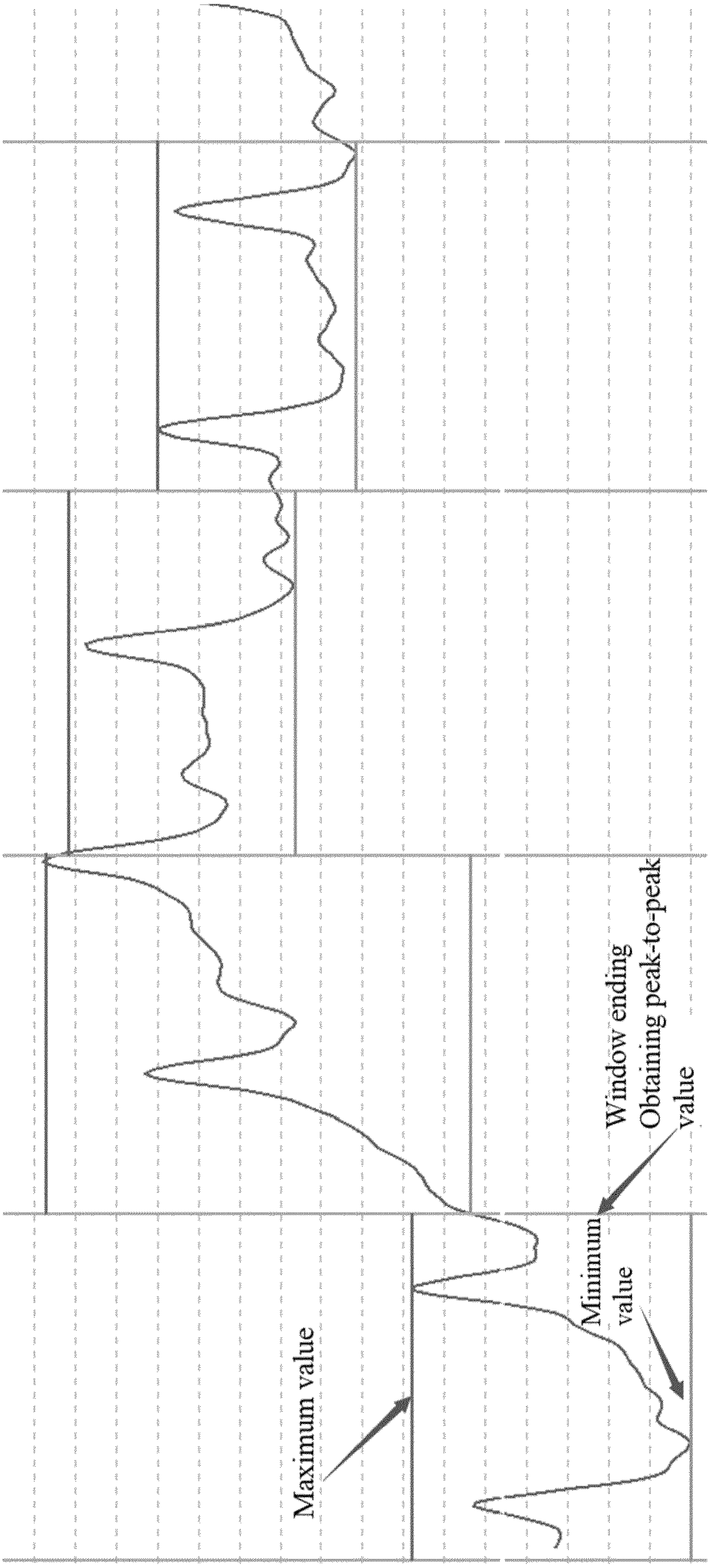
Maximum value
Minimum value
Window ending
Obtaining peak-to-peak value

FIG. 1

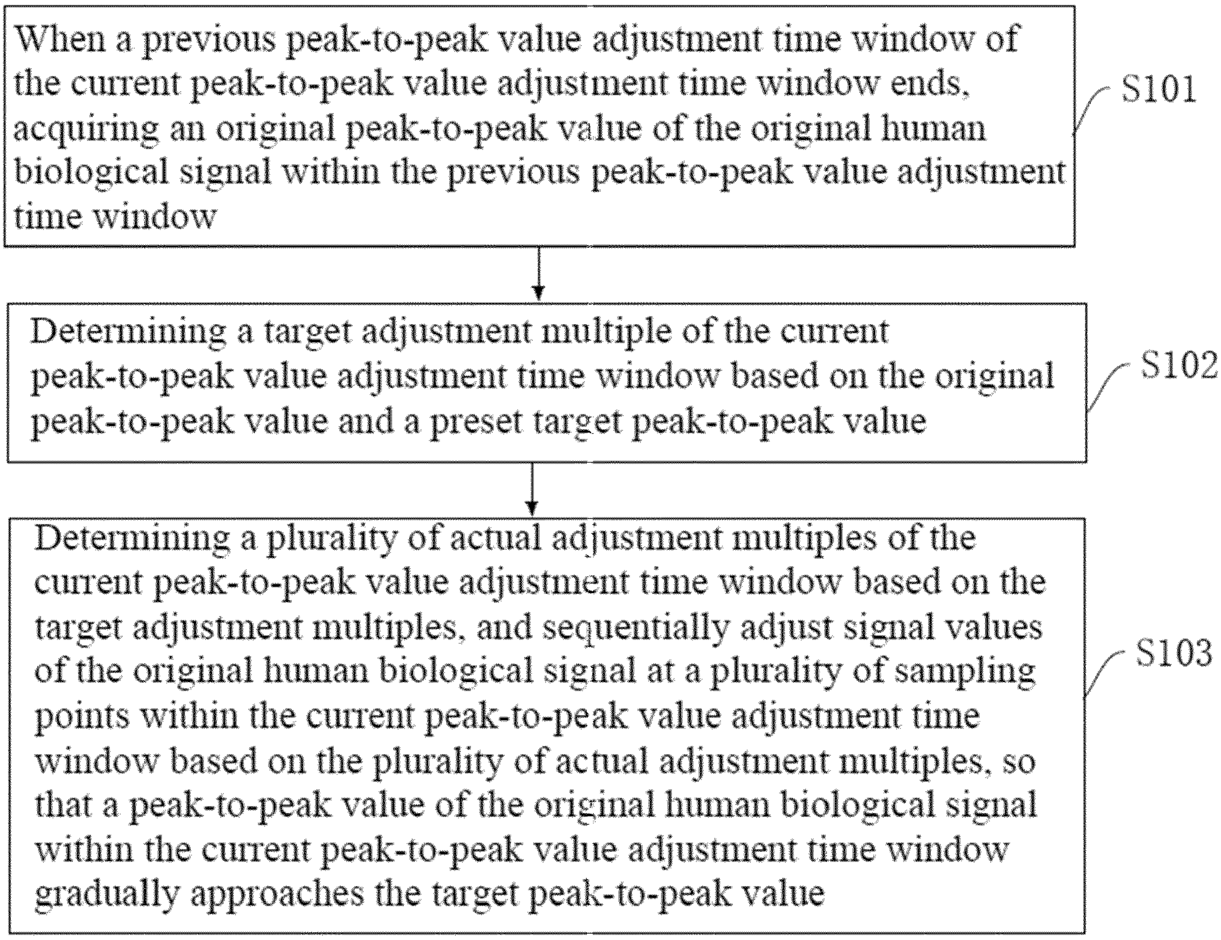
When a previous peak-to-peak value adjustment time window of the current peak-to-peak value adjustment time window ends, acquiring an original peak-to-peak value of the original human biological signal within the previous peak-to-peak value adjustment time window
S101
Determining a target adjustment multiple of the current peak-to-peak value adjustment time window based on the original peak-to-peak value and a preset target peak-to-peak value
S102
Determining a plurality of actual adjustment multiples of the current peak-to-peak value adjustment time window based on the target adjustment multiples, and sequentially adjust signal values of the original human biological signal at a plurality of sampling points within the current peak-to-peak value adjustment time window based on the plurality of actual adjustment multiples, so that a peak-to-peak value of the original human biological signal within the current peak-to-peak value adjustment time window gradually approaches the target peak-to-peak value
S103

FIG. 2

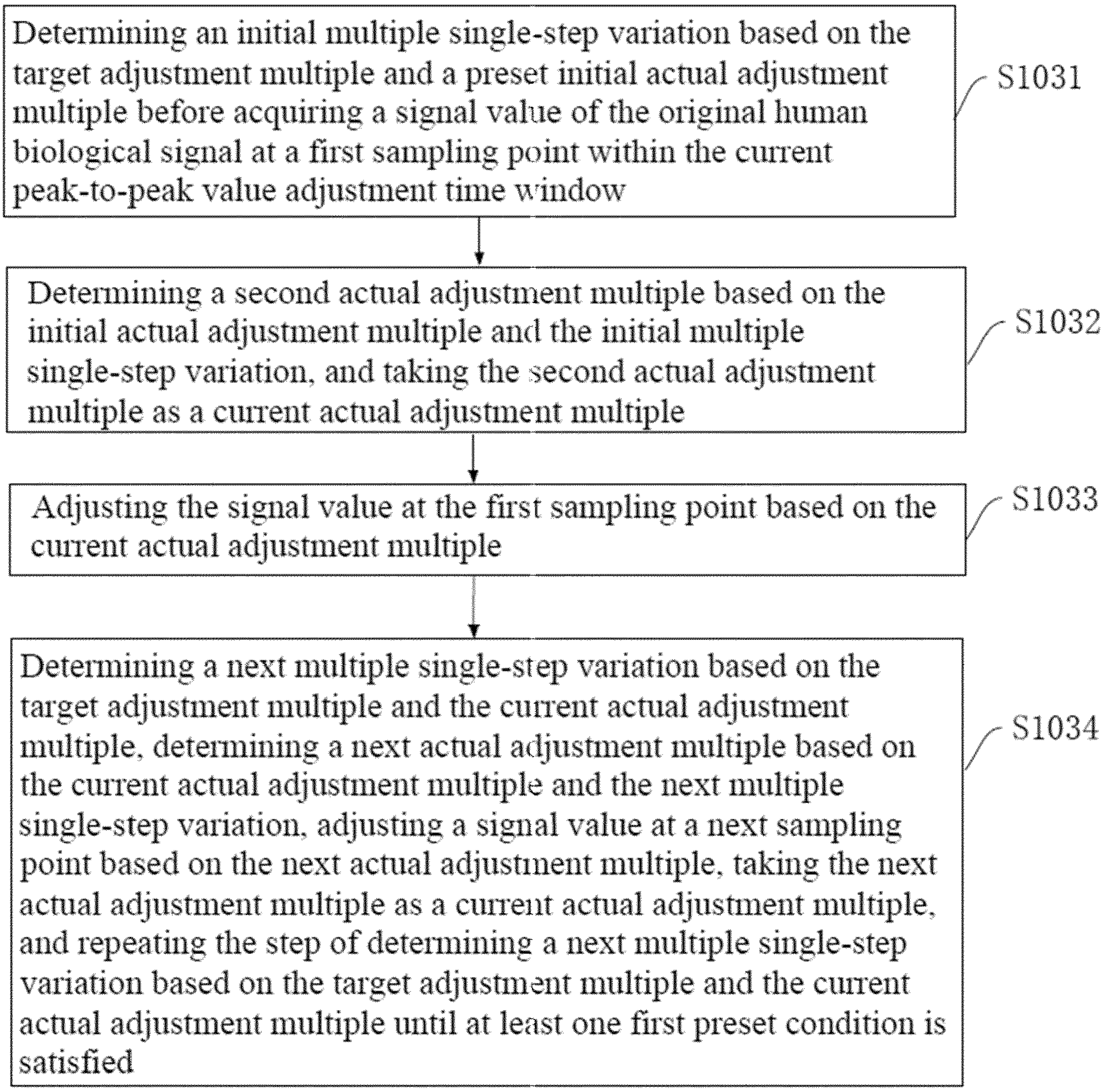
Determining an initial multiple single-step variation based on the target adjustment multiple and a preset initial actual adjustment multiple before acquiring a signal value of the original human biological signal at a first sampling point within the current peak-to-peak value adjustment time window
S1031
Determining a second actual adjustment multiple based on the initial actual adjustment multiple and the initial multiple single-step variation, and taking the second actual adjustment multiple as a current actual adjustment multiple
S1032
Adjusting the signal value at the first sampling point based on the current actual adjustment multiple
S1033
Determining a next multiple single-step variation based on the target adjustment multiple and the current actual adjustment multiple, determining a next actual adjustment multiple based on the current actual adjustment multiple and the next multiple single-step variation, adjusting a signal value at a next sampling point based on the next actual adjustment multiple, taking the next actual adjustment multiple as a current actual adjustment multiple, and repeating the step of determining a next multiple single-step variation based on the target adjustment multiple and the current actual adjustment multiple until at least one first preset condition is satisfied
S1034

FIG. 3

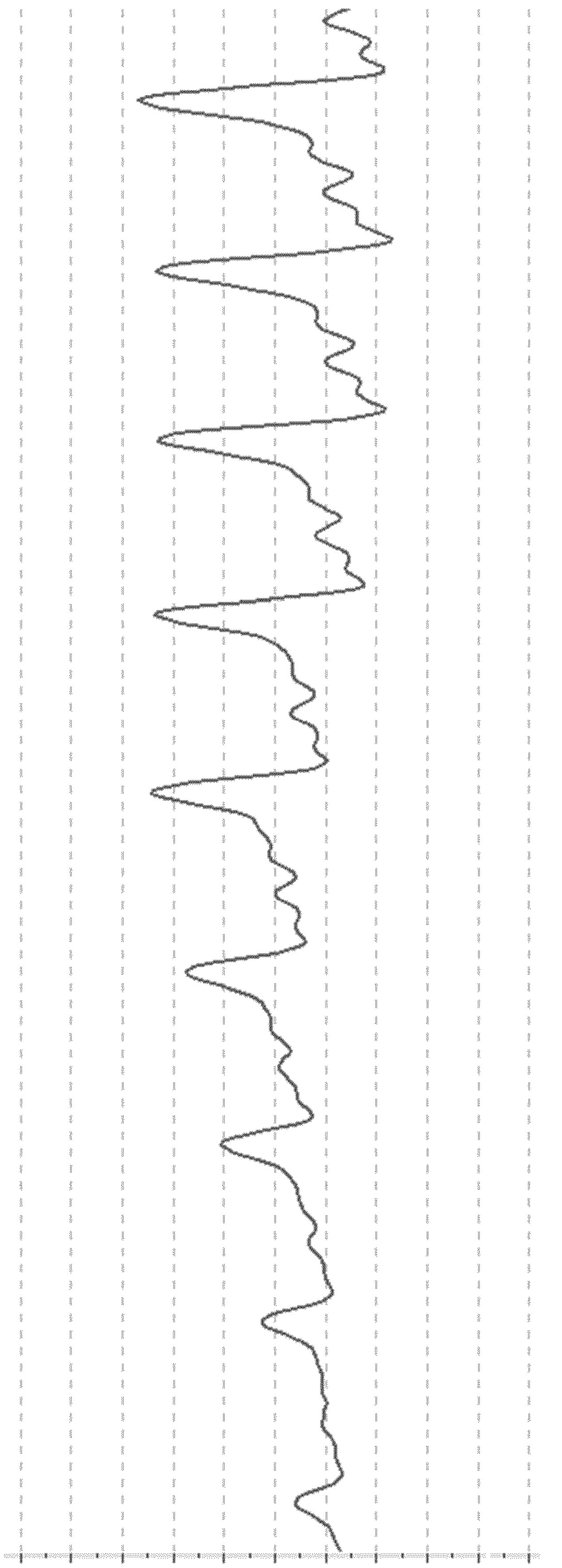


FIG. 4

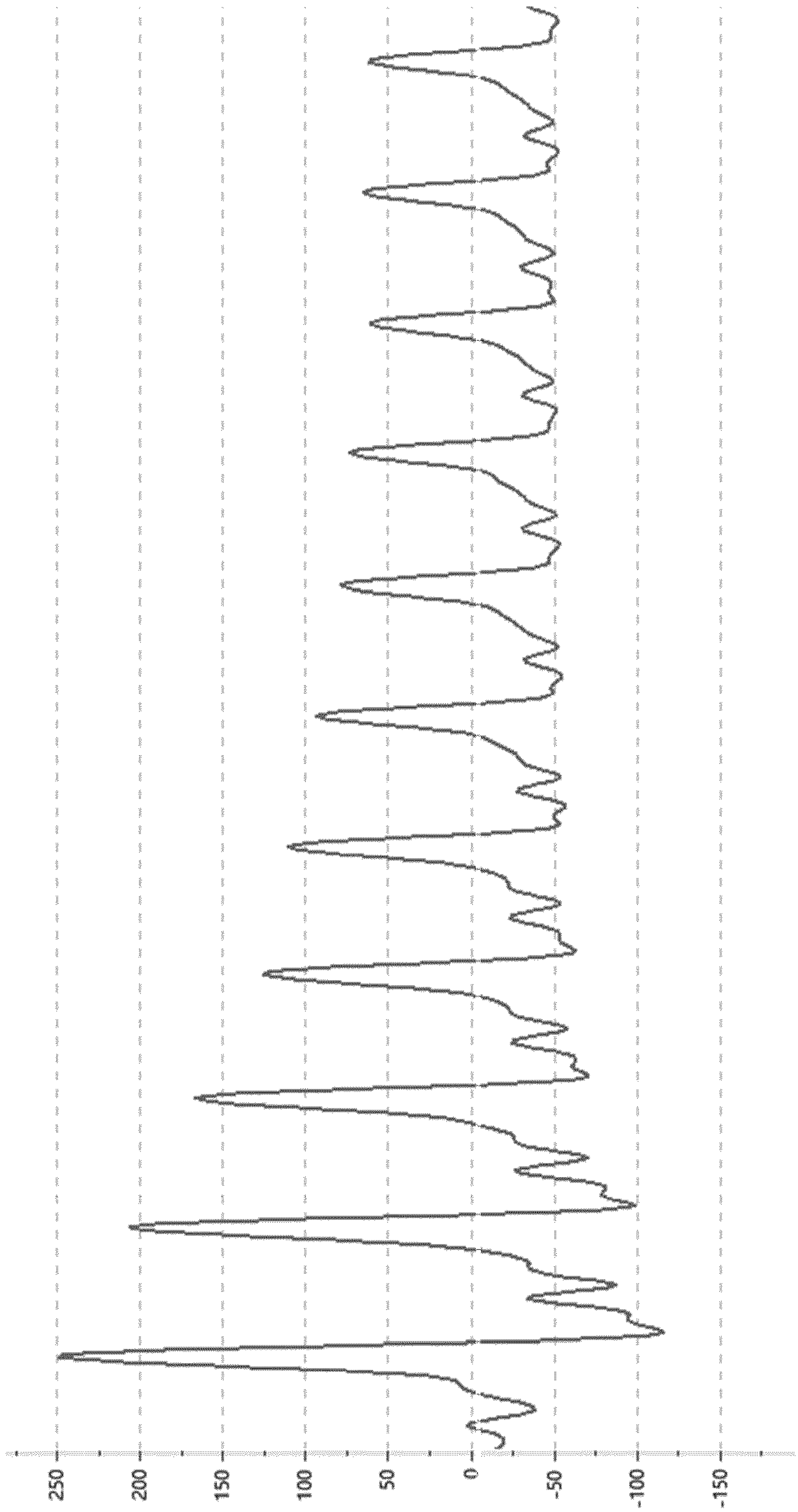


FIG. 5

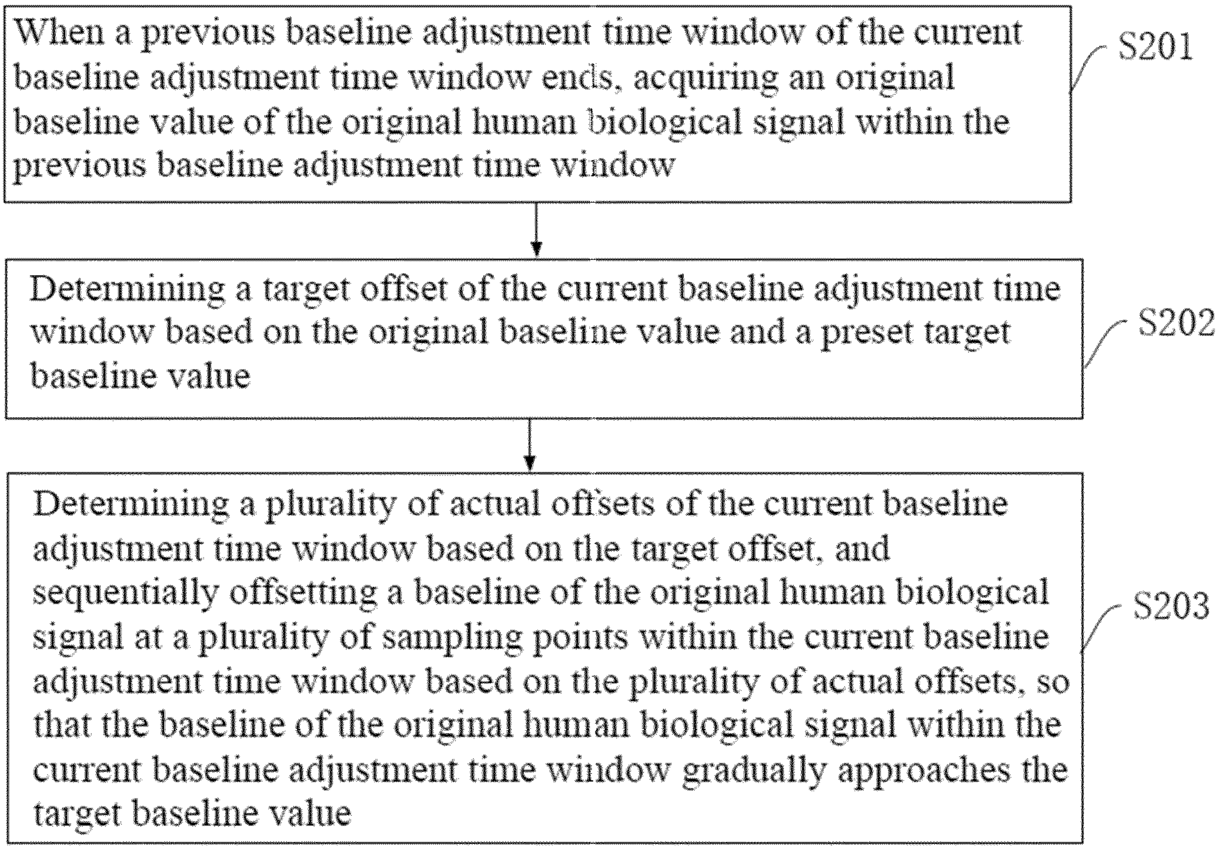
When a previous baseline adjustment time window of the current baseline adjustment time window ends, acquiring an original baseline value of the original human biological signal within the previous baseline adjustment time window
S201
Determining a target offset of the current baseline adjustment time window based on the original baseline value and a preset target baseline value
S202
Determining a plurality of actual offsets of the current baseline adjustment time window based on the target offset, and sequentially offsetting a baseline of the original human biological signal at a plurality of sampling points within the current baseline adjustment time window based on the plurality of actual offsets, so that the baseline of the original human biological signal within the current baseline adjustment time window gradually approaches the target baseline value
S203

FIG. 6

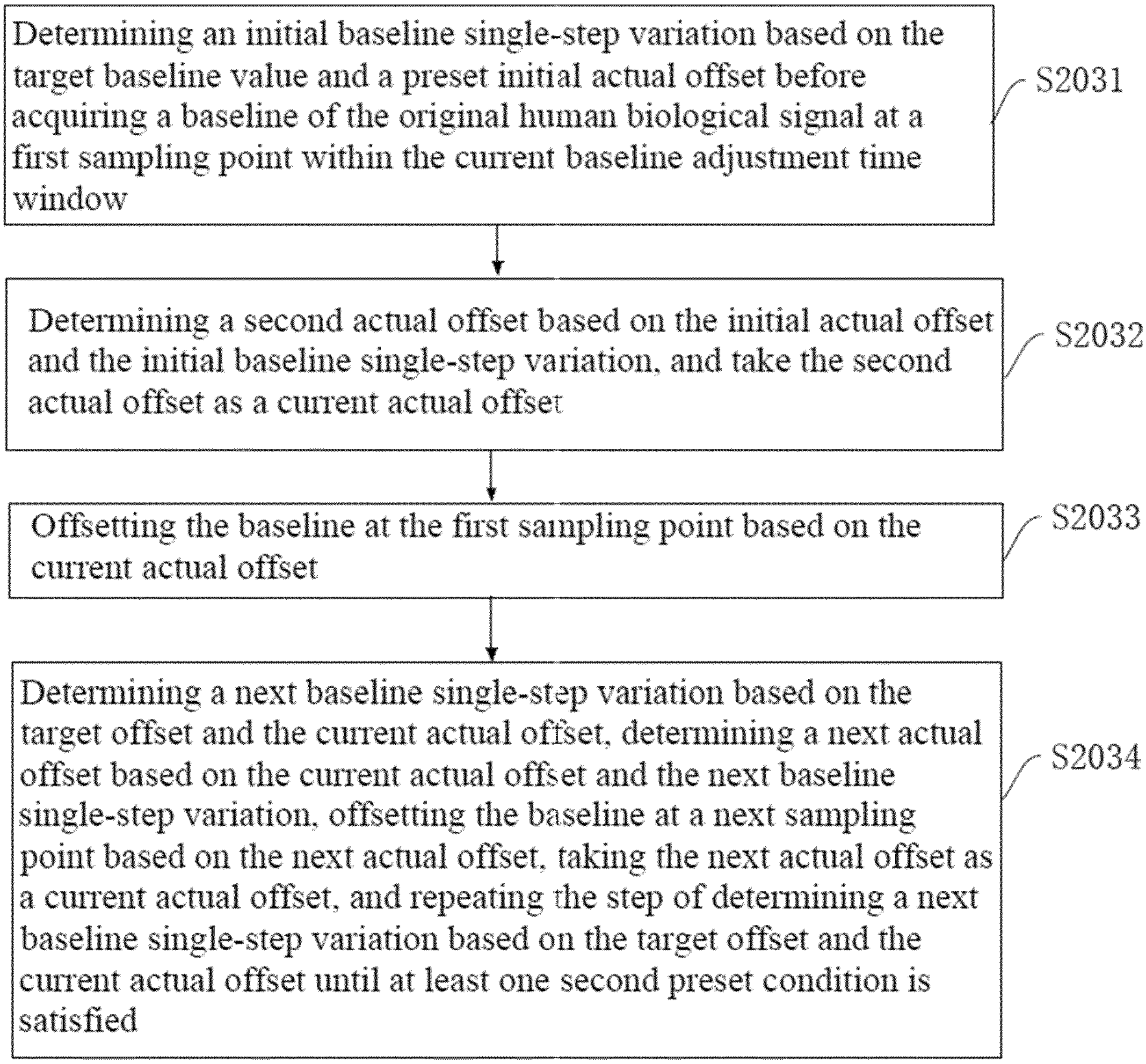
Determining an initial baseline single-step variation based on the target baseline value and a preset initial actual offset before acquiring a baseline of the original human biological signal at a first sampling point within the current baseline adjustment time window
S2031
Determining a second actual offset based on the initial actual offset and the initial baseline single-step variation, and take the second actual offset as a current actual offset
S2032
Offsetting the baseline at the first sampling point based on the current actual offset
S2033
Determining a next baseline single-step variation based on the target offset and the current actual offset, determining a next actual offset based on the current actual offset and the next baseline single-step variation, offsetting the baseline at a next sampling point based on the next actual offset, taking the next actual offset as a current actual offset, and repeating the step of determining a next baseline single-step variation based on the target offset and the current actual offset until at least one second preset condition is satisfied
S2034

FIG. 7

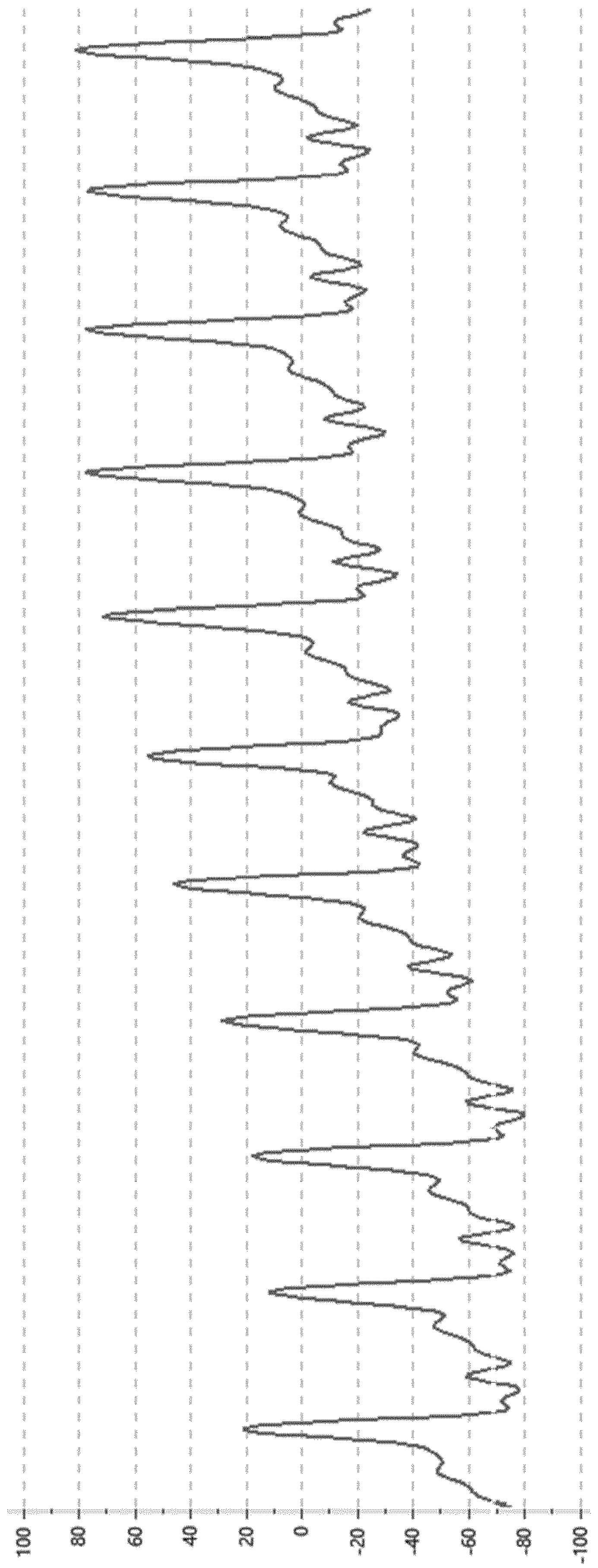


FIG. 8

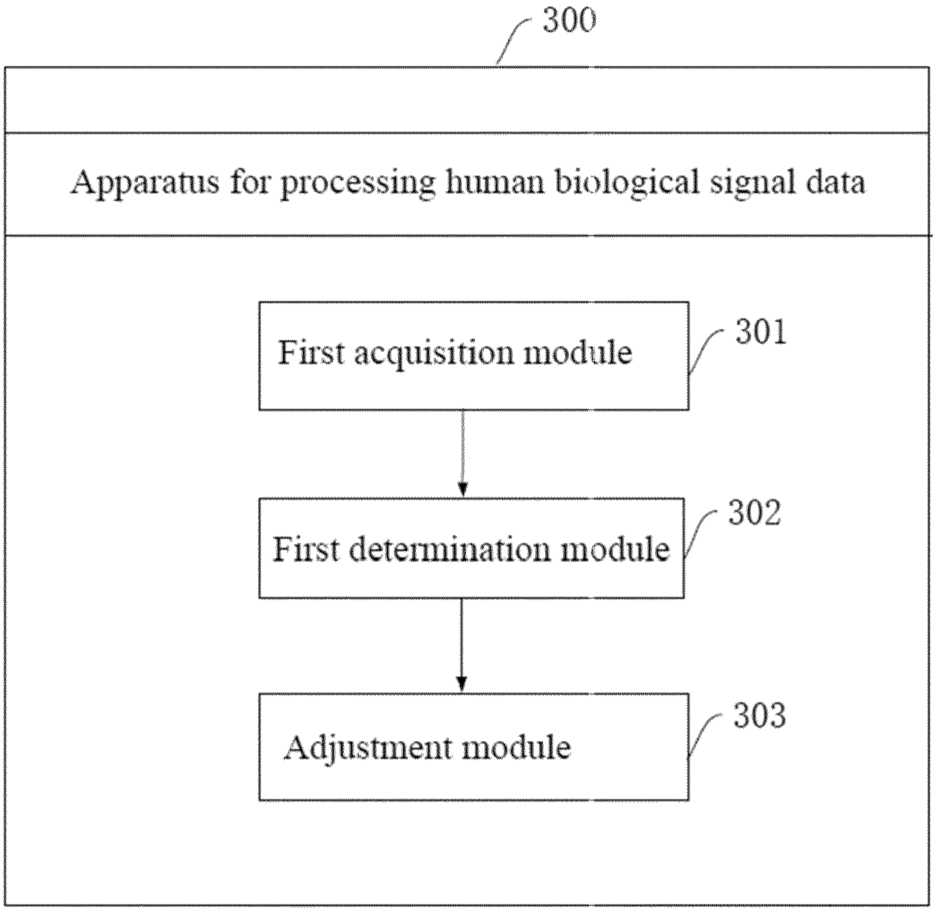
300
Apparatus for processing human biological signal data
First acquisition module
301
First determination module
302
Adjustment module
303

FIG. 9

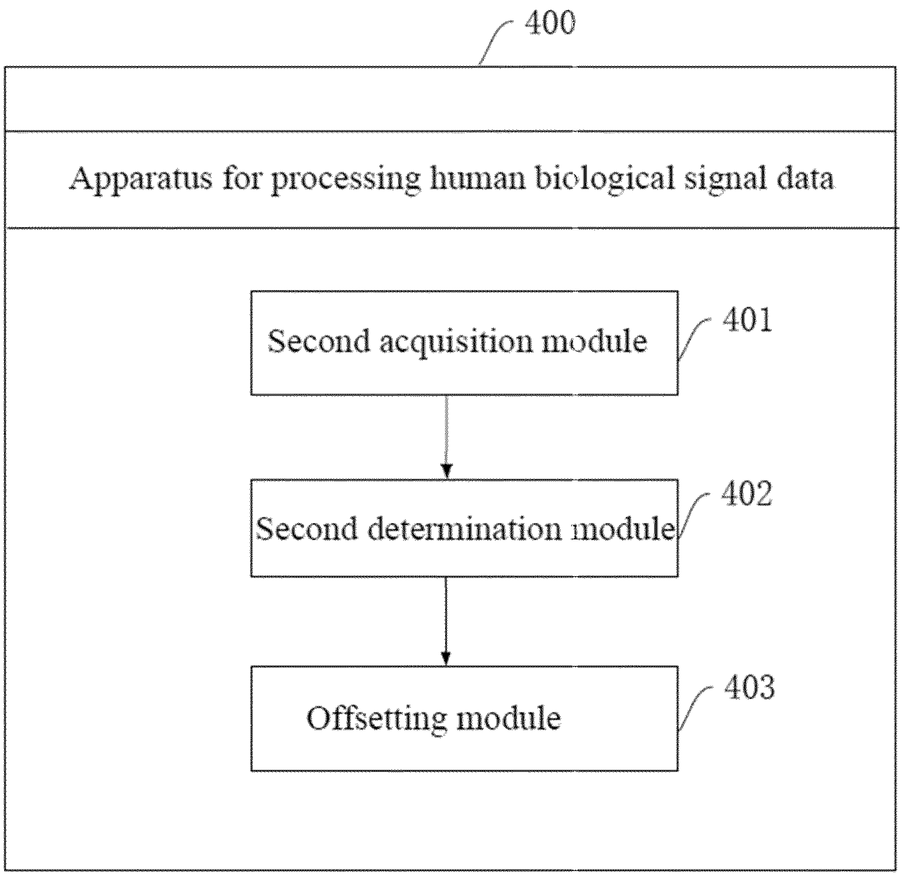
400
Apparatus for processing human biological signal data
Second acquisition module
401
Second determination module
402
Offsetting module
403

FIG. 10

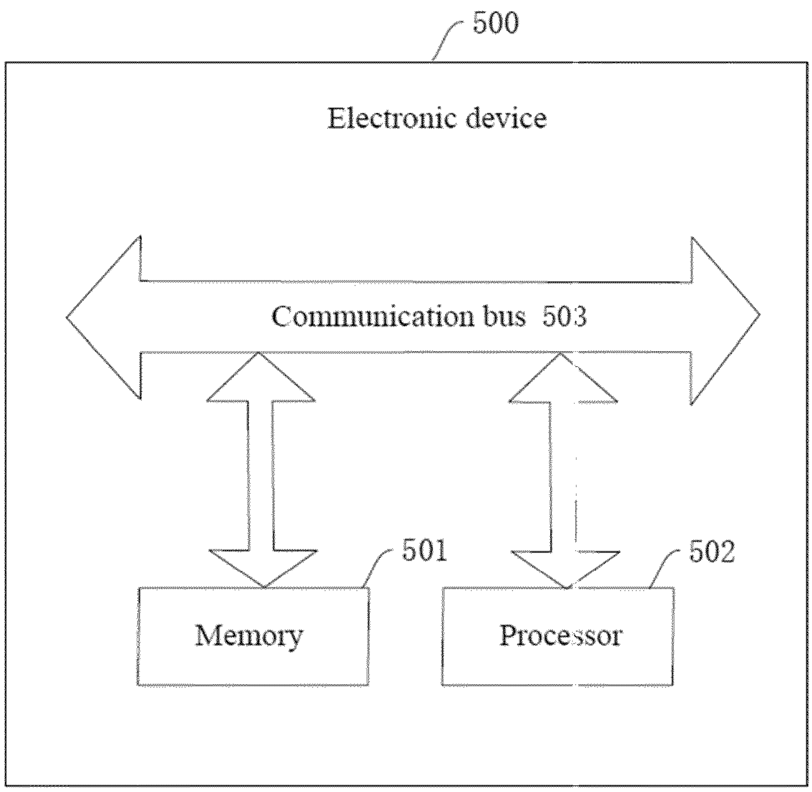
500
Electronic device
Communication bus 503
501
502
Memory
Processor

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101534102 A